# FASCICULE DE BREVET EUROPEEN

(11) **EP 1 064 919 B1**
(45) Date de publication et mention de la délivrance du brevet: **19.11.2003**
(21) Numéro de dépôt: 00401662.2
(22) Date de dépôt: 13.06.2000
(51) Int. Cl.: A61K 7/06, A61K 7/48, A61K 7/032

(54) **Mascara comprenant des polymères filmogènes**
Mascara enthaltend filmbildende Polymere
Mascara comprising film-forming polymers

(30) Priorité: 30.06.1999 FR 9908412
(43) Date de publication de la demande: 03.01.2001
(73) Titulaire: L'OREAL, 75008 Paris (FR)
(72) Inventeur: Bodelin, Sophie, 92170 Vanves (FR)
(74) Mandataire: Kromer, Christophe

(56) Documents cités:
- WO-A-95/34271
- WO-A-98/19653
- DE-A- 19 750 520
- FR-A- 2 528 699
- FR-A- 2 739 288
- GB-A- 1 110 240
- US-A- 5 534 247
- CHEMICAL ABSTRACTS, vol. 131, no. 22, 29 novembre 1999 (1999-11-29) Columbus, Ohio, US; abstract no. 303229, OKAJIMA, TAKAO ET AL: "Temporary hair-coloring compositions" XP002135146 & JP 11 292744 A (KAO CORP., JAPAN) 26 octobre 1999 (1999-10-26)

## Description

La présente invention concerne une composition de revêtement des fibres kératiniques comprenant un mélange de polymères filmogènes. L'invention se rapporte également à l'utilisation de cette composition pour le maquillage des fibres kératiniques, ainsi qu'à un procédé de maquillage de ces dernières. La composition et le procédé de maquillage selon l'invention sont plus particulièrement destinés aux fibres kératiniques sensiblement longitudinales d'êtres humains telles que les cils, les sourcils et les cheveux, y compris les faux-cils et les postiches. La composition peut être une composition de maquillage, une base de maquillage, une composition à appliquer sur un maquillage, dite encore top-coat, ou bien encore une composition de traitement cosmétique des fibres kératiniques. Plus spécialement, l'invention porte sur un mascara.

Les compositions de revêtement des cils, appelées mascara, comprennent généralement, de façon connue, au moins une cire et au moins un polymère filmogène pour déposer un film de maquillage sur les cils et gainer ces derniers, comme le décrit par exemple le document WO-A-95/15741. Les utilisatrices attendent pour ces produits de bonnes propriétés cosmétiques telles que l'adhérence sur les cils, un allongement ou un recourbement des cils, ou bien encore une bonne tenue du mascara dans le temps, notamment une bonne résistance aux frottements par exemple des doigts ou des tissus (mouchoirs, serviettes).

Pour conférer un effet d'allongement aux cils, le document FR-A-2528699 propose un mascara comprenant des cires et une association de polymère anionique et de polymère cationique. Toutefois, ces compositions ne permettent pas d'obtenir un bon recourbement des cils.

Le but de la présente invention est de disposer d'une composition de maquillage des fibres kératiniques, et notamment des fibres tels que les cils, s'appliquant facilement et conférant un bon recourbement des fibres kératiniques.

Les inventeurs ont découverts qu'une telle composition pouvait être obtenue en utilisant une association particulière de polymères filmogènes.

Plus précisément, l'invention a pour objet une composition de maquillage des fibres kératiniques comprenant au moins un polymère filmogène comprenant au moins un polymère filmogène cationique et au moins un polymère filmogène anionique caractérisé par le fait qu'elle comprend une dispersion aqueuse de polymère filmogène non-ionique qui est un copolymère d'acrylate d'éthyle et de méthacrylate de méthyle.

La composition selon l'invention s'applique facilement et s'accroche bien sur les fibres kératiniques tels que les cils. On constate que les résultats de maquillage comme le gainage, l'allongement et le recourbement des cils sont obtenus rapidement et facilement après application sur les cils. Le maquillage est confortable pour l'utilisatrice. Le maquillage s'élimine facilement avec les démaquillants classiques.

L'invention a aussi pour objet un procédé de revêtement des fibres kératiniques, notamment des cils, consistant à appliquer sur les fibres kératiniques une composition telle que définie précédemment.

L'invention a également pour objet l'utilisation d'une composition telle que définie précédemment pour recourber et/ou allonger et/ou gainer les cils et/ou pour obtenir un maquillage rapide et/ou facile à appliquer et/ou de bonne tenue.

On entend par polymère filmogène un polymère conduisant seul, ou en présence d'agent plastifiant, à un film isolable.

Le document US-A-5 534 247 décrit des mascaras comportant un polymère fixant tel un polymère ou copolymère d'alkyl acrylate dont l'alkyl est en C₄ à C₁₈, l'alkyl méthacrylate ayant un alkyl en C₁-C₄.

La composition selon l'invention comprend une dispersion aqueuse d'un polymère filmogène non-ionique (qui est un copolymère d'acrylate d'éthyle et de méthacrylate de méthyle tel que celui vendu sous la dénomination "DAITOSOL 5000 AD" par la société DAITO KASEY KOGYO.

La dispersion aqueuse de copolymère d'acrylate d'éthyle et de méthacrylate de méthyle peut comprendre des particules de polymère ayant une taille allant de 10 à 500 nm, et de préférence de 20 à 300 nm.

Par dispersion aqueuse de polymère, généralement connue sous le nom de latex ou pseudolatex, on entend une phase contenant de l'eau et éventuellement un composé soluble dans l'eau, dans laquelle est dispersé directement le polymère sous forme de particules.

En pratique, le copolymère d'acrylate d'éthyle et de méthacrylate de méthyle peut être présent dans la composition selon l'invention en une teneur en matières sèches allant de 0,1 % à 15 % en poids, par rapport au poids total de la composition, et mieux de 0,5 % à 8 % en poids.

Selon l'invention, la composition peut contenir tout polymère anionique, de préférence non réticulé, ou cationique connu en soi.

Ces polymères peuvent être utilisés sous forme solubilisée ou sous forme de dispersions aqueuses de particules solides de polymère.

Les polymères anioniques généralement utilisés peuvent être des polymères comportant des groupements dérivés d'acide carboxylique, sulfonique ou phosphorique et peuvent avoir un poids moléculaire moyen en poids compris entre environ 500 et 5.000.000.

1) Les groupements carboxyliques peuvent être apportés par des monomères mono ou diacides carboxyliques insaturés tels que ceux répondant à la formule (I) suivante : 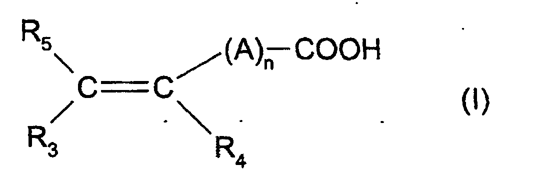 dans laquelle n est un nombre entier de 0 à 10, A désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₅ désigne un atome d'hydrogène, un groupement phényle ou benzyle, R₃ désigne un atome d'hydrogène, un groupement alkyle inférieur ou carboxyle, R₄ désigne un atome d'hydrogène, un groupement alkyle inférieur, un groupement -CH₂-COOH, phényle ou benzyle ;

Dans la formule précitée un radical alkyle inférieur désigne de préférence un groupement ayant 1 à 4 atomes de carbone et en particulier, méthyle et éthyle.

Les polymères anioniques à groupements carboxyliques préférés selon l'invention sont :
A) les homo- ou copolymères d'acide acrylique ou méthacrylique ou leurs sels (notamment sels de métaux alcalins ou alcalino-terreux, d'ammoniums) et en particulier les produits commercialisés sous les dénominations VERSICOL E ou K par la société ALLIED COLLOID, ULTRAHOLD par la société BASF, DARVAN 7 par la société VANDERBILT. Les copolymères d'acide acrylique et d'acrylamide commercialisés sous la forme de leur sel de sodium sous les dénominations RETEN 421, 423 ou 425 par la Société HERCULES, les sels de sodium des acides polyhydroxycarboxyliques.
B) les copolymères des acides acrylique ou méthacrylique avec un monomère monoéthylénique tel que l'éthylène, le styrène, les esters vinyliques, les esters d'acide acrylique ou méthacrylique. Ces copolymères peuvent être greffés sur un polyalkylène glycol tel que le polyéthylène glycol. De tels polymères sont décrits en particulier dans le brevet français 1.222.944 et la demande allemande 2.330.956. On peut notamment citer les copolymères comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER par la Société AMERICAN CYANAMID. On peut également citer les copolymères d'acide acrylique et de méthacrylate d'alkyle en C₁-C₄ et les terpolymères de vinylpyrrolidone, d'acide (méth)acrylique et de (méth)acrylate d'alkyle en C₁-C₂₀ par exemple de lauryle (tel que celui commercialisé par la société ISP sous la dénomination ACRYLIDONE LM), de tertiobutyle (LUVIFLEX VBM 70 commercialisé par BASF) ou de méthyle (STEPANHOLD EXTRA commercialisé par STEPAN) et les terpolymères acide méthacrylique/ acrylate d'éthyle/ acrylate de tertiobutyle tel que le produit commercialisé sous la dénomination LUVIMER 100 P par la société BASE.
C) les copolymères dérivés d'acide crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés ou encore un ester vinylique, allylique ou méthallylique d'un acide carboxylique α- ou β-cyclique. De tels polymères sont décrits entre autres dans les brevets français 1.222.944, 1.580.545, 2.265.782, 2.265.781, 1.564.110 et 2.439.798. Des produits commerciaux entrant dans cette classe sont les résines 28-29-30, 26-13-14 et 28-13-10 commercialisées par la société NATIONAL STARCH.
D) les copolymères dérivés d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisis parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées ; De tels polymères sont décrits en particulier dans les brevets US 2.047.398, 2.723.248, 2.102.113, le brevet GB 839.805 et notamment ceux commercialisés sous les dénominations GANTREZ AN ou ES, AVANTAGE CP par la société ISP.
   - les copolymères comprenant (i) un ou plusieurs anhydrides maléique, citraconique, itaconique et (ii) un ou plusieurs monomères choisis parmi les esters allyliques ou méthallyliques comportant éventuellement un ou plusieurs groupement acrylamide, méthacrylamide, α-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.

   Ces polymères sont par exemple décrits dans les brevets français 2.350.384 et 2.357.241 de la demanderesse.
E) les polyacrylamides comportant des groupements carboxylates,
   - et leurs mélanges.

2) Les polymères comprenant les groupements sulfoniques peuvent être des polymères comportant des motifs vinylsulfonique, styrène sulfonique, naphtalène sulfonique, acrylamido alkylsulfonique, ou bien encore des polyesters sulfoniques.

Ces polymères peuvent être notamment choisis parmi :
- les sels de l'acide polyvinylsulfonique ayant un poids moléculaire moyen en poids compris entre environ 1.000 et 100.000 ainsi que les copolymères avec un comonomère insaturé tel que les acides acrylique ou méthacrylique et leurs esters ainsi que l'acrylamide ou ses dérivés, les éthers vinyliques et la vinylpyrrolidone ;
- les sels de l'acide polystyrène sulfonique les sels de sodium ayant un poids moléculaire moyen en poids d'environ 500.000 et d'environ 100.000 commercialisés respectivement sous les dénominations Flexan 500 et Flexan 130 par National Starch. Ces composés sont décrits dans le brevet FR 2.198.719 ;
- les sels d'acides polyacrylamide sulfoniques ceux mentionnés dans le brevet US 4.128.631 et plus particulièrement l'acide polyacrylamidoéthylpropane sulfonique commercialisé sous la dénomination COSMEDIA POLYMER HSP 1180 par Henkel ;
- les polyesters sulfoniques portant au moins un groupement -SO₃M avec M représentant un atome d'hydrogène, un ion ammonium NH₄⁺ ou un ion métallique. Le copolyester peut être par exemple un copolymère d'au moins un diacide carboxylique, d'au moins un diol et d'au moins un monomère aromatique bifonctionnel portant un groupement -SO₃M avec M représentant un atome d'hydrogène, un ion ammonium NH₄⁺ ou un ion métallique.

L'acide dicarboxylique peut être choisi parmi l'acide phtalique, l'acide isophtalique, l'acide téréphtalique. Le diol peut être choisi parmi l'éthylène glycol, le diéthylène glycol, le triéthylène glycol, le 1,3-propanediol, le 1,4-cyclohexane diméthanol, le 1,4-butanediol. Le monomère aromatique bifonctionnel portant le groupement -SO₃M peut être choisi parmi l'acide sulfoisophtalique, notamment le sel de sodium de l'acide 5-sulfo-isophtalique, l'acide sulfotéréphtalique, l'acide sulfophtalique, l'acide 4-sulfonaphtalène-2,7-dicarboxylique.

Comme polyester préféré, on peut utiliser un polyester consistant essentiellement en des unités répétées d'acide isophtalique, de diol et d'acide sulfo-isophtalique, et notament les sulfopolyesters obtenus par condensation de di-éthylèneglycol, de cyclohexane di-méthanol, d'acide isophtalique, d'acide sulfoisophtalique. Comme polyester sulfonique, on peut utiliser ceux commercialisés sous les dénominations AQ55S, AQ 38S, AQ 29S par la société EASTMAN.

On peut également employer comme polymère anionique l'acide (désoxy)ribonucléique.

Selon l'invention, les polymères anioniques sont de préférence choisis parmi les copolymères d'acide acrylique tels que les terpolymères acide acrylique / acrylate d'éthyle / N-tertiobutylacrylamide commercialisés notamment sous la dénomination ULTRAHOLD STRONG par la société BASF, les copolymères dérivés d'acide crotonique tels que les terpolymères acétate de vinyle / tertio-butyl benzoate de vinyle / acide crotonique et les terpolymères acide crotonique / acétate de vinyle/néododécanoate de vinyle commercialisés notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique mono estérifiés commercialisés par exemple sous la dénomination GANTREZ par la société ISP, les copolymères d'acide méthacrylique et de méthacrylate de méthyle commercialisés sous la dénomination EUDRAGIT L par la société ROHM PHARMA, les copolymères d'acide méthacrylique/ méthacrylate de méthyle / acrylate d'alkyle en C1-C4 / acide acrylique ou méthacrylate d'hydroxyalkyle en C1-C4 commercialisés sous forme de dispersions sous la dénomination AMERHOLD DR 25 par la société AMERCHOL ou sous la dénomination ACUDYNE 255 par la société ROHM & HAAS, les copolymères d'acide méthacrylique et d'acrylate d'éthyle commercialisés sous la dénomination LUVIMER MAEX ou MAE par la société BASF et les copolymères acétate de vinyle/acide crotonique, les copolymères acétate de vinyle/acide crotonique greffés par du polyéthylèneglycol sous la dénomination ARISTOFLEX A par la société BASF, les homopolymères d'acide acrylique ou méthacrylique commercialisés par exemple sous la dénomination VERSICOL E 5 ou le polyméthacrylate de sodium vendu sous la dénomination DARVAN 7 par la société VANDERBILT, et leurs mélanges.

Les polymères anioniques les plus particulièrement préférés sont choisis parmi les polymères anioniques non réticulés comme les copolymères méthylvinyléther / anhydride maléique mono estérifiés commercialisés sous la dénomination GANTREZ ES 425 par la société ISP, les terpolymères acide acrylique / acrylate d'éthyle / N-tertiobutylacrylamide commercialisés sous la dénomination ULTRAHOLD STRONG par la société BASF, les copolymères d'acide méthacrylique et de méthacrylate de méthyle commercialisés sous la dénomination EUDRAGIT L par la société ROHM PHARMA, les terpolymères acétate de vinyle / tertio-butyl benzoate de vinyle / acide crotonique et les terpolymères acide crotonique / acétate de vinyle / néododécanoate de vinyle commercialisés sous la dénomination Résine 28-29-30 par la société NATIONAL STARCH, les copolymères d'acide méthacrylique et d'acrylate d'éthyle commercialisés sous la dénomination LUVIMER MAEX OU MAE par la société BASF, les terpolymères vinylpyrrolidone / acide acrylique / méthacrylate de lauryle commercialisés sous la dénomination ACRYLIDONE LM par la société ISP et les homopolymères d'acide acrylique ou méthacrylique commercialisés par exemple sous la dénomination VERSICOL E 5 OU le polyméthacrylate de sodium vendu sous la dénomination DARVAN 7 par la société VANDERBILT, et leurs mélanges.

Selon l'invention, on peut également utiliser des polymères anioniques sous forme de latex ou de pseudolatex, c'est à dire sous forme d'une dispersion de particules de polymères insolubles.

3) Selon l'invention, on peut également utiliser les polymères anioniques de type siliconés greffés comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-0 412 704, EP-A-0 412 707, EP-A-0 640 105 et WO 95/00578, EP-A-0582 152 et WO 93/23009 et les brevets US 4,693,935, US 4,728,571 et US 4,972,037.

De tels polymères sont par exemple les copolymères susceptibles d'être obtenus par polymérisation radicalaire à partir du mélange de monomères constitué par :
a) 50 à 90% en poids d'acrylate de tertiobutyle ;
b) 1 à 40% en poids d'acide acrylique ;
c) 5 à 40% en poids de macromère siliconé de formule (II): 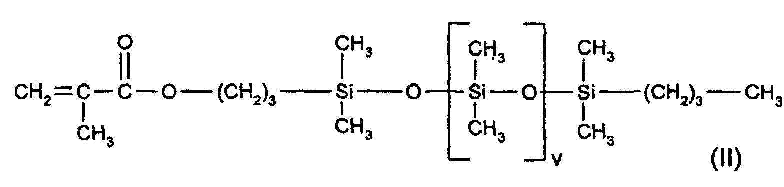 avec v étant un nombre allant de 5 à 700 ; les pourcentages en poids étant calculés par rapport au poids total des monomères.

Une famille de polymères siliconés à squelette polysiloxanique greffé par des monomères organiques non siliconés convenant particulièrement bien à la mise en oeuvre de la présente invention est constituée par les polymères siliconés comportant dans leur structure le motif de formule (III) suivant : 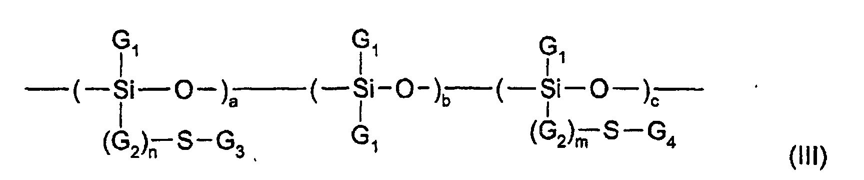 dans lequel les radicaux G₁ identiques ou différents, représentent l'hydrogène ou un radical alkyle en C₁-C₁₀ ou encore un radical phényle ; les radicaux G₂, identiques ou différents, représentent représente un groupe alkylène en C₁-C₁₀ ; G₃ représente un reste polymérique résultant de l'(homo)polymérisation d'au moins un monomère anionique à insaturation éthylénique ; G₄ représente un reste polymérique résultant de l'(homo)polymérisation d'au moins un monomère d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

De préférence, le motif de formule (III) ci-dessus présente au moins l'une, et encore plus préférentiellement l'ensemble, des caractéristiques suivantes :
- les radicaux G, désignent un radical alkyle en C₁-C₁₀ de préférence le radical méthyle ;
- n est non nul, et les radicaux G₂ représentent un radical divalent en C₁-C₃, de préférence un radical propylène ;
- G₃ représente un radical polymérique résultant de l'(homo)polymérisation d'au moins un monomère du type acide carboxylique à insaturation éthylénique, de préférence l'acide acrylique et/ou l'acide méthacrylique ;
- G₄ représente un radical polymérique résultant de l'(homo)polymérisation d'au moins un monomère du type (méth)acrylate d'alkyle en C₁-C₁₀ , de préférence le (méth)acrylate d'isobutyle ou de méthyle.

De préférence, le motif de formule (III) ci-dessus peut également présenter l'ensemble des caractéristiques suivantes :
- les radicaux G₁ désignent un radical alkyle, de préférence le radical méthyle ;
- n est non nul, et les radicaux G₂ représentent un radical divalent en C₁-C₃, de préférence un radical propylène ;
- G₃ représente un radical polymérique résultant de l'(homo)polymérisation d'au moins un monomère du type acide carboxylique à insaturation éthylénique, de préférence l'acide acrylique et/ou l'acide méthacrylique ;
- c est égal zéro.

Des exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle tel que le poly-(méth)acrylate d'isobutyle.
On utilise particulièrement les polymères siliconés greffés de formule (III) de structure polyméthyl/méthylsiloxane à groupements propyl thio-3 acide polyméthacrylique et groupements propyl thio-3 polyméthacrylate de méthyle et les polymères siliconés greffés de formule (III) de structure polyméthyl/méthylsiloxane à groupements propyl thio-3 acide polyacrylique.

Selon l'invention, le ou les polymères anioniques peuvent être présents en une teneur allant de 0,01 % à 20 % en poids, de préférence de 0,05 % à 15 % en poids, et encore plus préférentiellement de 0,1 % à 7 % en poids, du poids total de la composition.

Les polymères cationiques utilisables conformément à la présente invention peuvent être choisis parmi tous ceux déjà connus en soi notamment ceux décrits dans la demande de brevet EP-A- 0 337 354 et dans les demandes de brevets français FR-A- 2 270 846, 2 383 660, 2 598 611, 2 470 596 et 2 519 863.

De manière encore plus générale, au sens de la présente invention, l'expression "polymère cationique" désigne tout polymère contenant des groupements cationiques ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Une famille de polymères cationiques est celle des polymères cationiques siliconés. Parmi ces polymères, on peut citer :

(a) les polymères siliconés répondant à la formule (IV) suivante :

R⁶ ₐG⁵ ₃₋ₐSi(OSiG⁶ ₂)ₙ-(OSiG⁷ _{b}R⁷ _{2-b})ₘ-O-SiG⁸ _{3-a'}-R⁸ _{a'} (IV)

dans laquelle :
G⁵, G⁶, G⁷ et G⁸, identiques ou différents, désignent un atome d'hydrogène, un groupement phényle, OH, alkyle en C₁-C₁₈, par exemple méthyle, alcényle en C₂-C_{18,} ou alcoxy en C₁-C₁₈
a, a', identiques ou différents, désignent le nombre 0 ou un nombre entier de 1 à 3, en particulier 0,
b désigne 0 ou 1, et en particulier 1,
m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
R⁶, R⁷, R⁸, identiques ou différents, désignent un radical monovalent de formule -C_{q}H_{2q}Oₛ R⁹ₜL dans laquelle q est un nombre de 1 à 8, s et t, identiques ou différents, sont égaux à 0 ou à 1, R⁹ désigne un groupement alkylène éventuellement hydroxylé et L est un groupement aminé éventuellement quaternisé choisi parmi les groupements :

   - NR"-CH₂-CH₂-N'(R")₂

   -N(R")₂

   - N^{⊕}(R")₃A⁻

   - N^{⊕}H(R")₂A⁻

   - N^{⊕}H₂(R")A⁻

   - -N(R")-CH₂-CH₂-N^{⊕}R" H₂ A⁻,
dans lesquels R" peut désigner hydrogène, phényle, benzyle, ou un radical hydrocarboné saturé monovalent, par exemple un radical alkyle ayant de 1 à 20 atomes de carbone et A⁻ représente un ion halogénure tel que par exemple fluorure, chlorure, bromure ou iodure.

Des produits correspondant à cette définition sont par exemple les polysiloxanes dénommés dans le dictionnaire CTFA "amodiméthicone" et répondant à la formule (V) suivante : 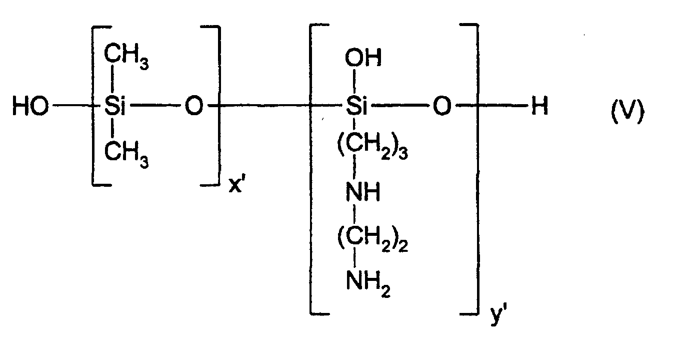 dans laquelle x' et y' sont des nombres entiers dépendant du poids moléculaire, généralement tels que ledit poids moléculaire est compris entre 5 000 et 20 000 environ.

Un produit correspondant à la formule (IV) est le polymère dénommé dans le dictionnaire CTFA "triméthylsilylamodiméthicone", répondant à la formule (VI) : 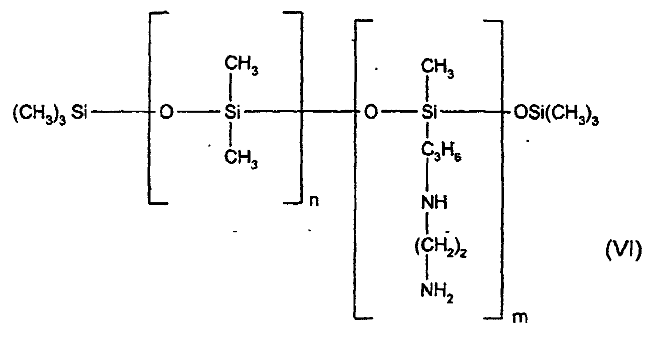 dans laquelle n et m ont les significations données ci-dessus pour la formule (IV).

Un produit commercial répondant à cette définition est un mélange (90/10 en poids) d'un polydiméthylsiloxane à groupements aminoéthyl aminoisobutyle et d'un polydiméthylsiloxane commercialisé sous la dénomination Q2-8220 par la société DOW CORNING.

De tels polymères sont décrits par exemple dans la demande de brevet EP-A-95238.

D'autres polymères répondant à la formule (IV) sont les polymères siliconés répondant à la formule suivante (VII) : 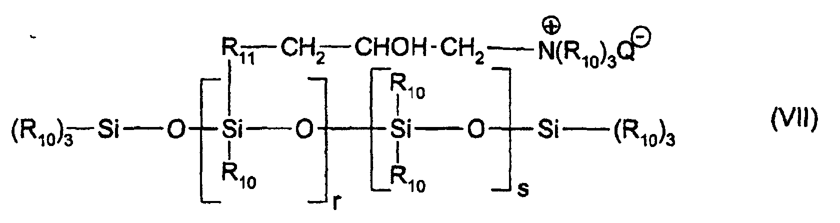 dans laquelle :
R₁₀ représente un radical hydrocarboné monovalent ayant de 1 à 18 atomes de carbone, et en particulier un radical alkyle en C₁-C₁₈, ou alcényle en C₂-C₁₈, par exemple méthyle ;
R₁₁ représente un radical hydrocarboné divalent, notamment un radical alkylène en C₁-C₁₈ ou un radical alkylèneoxy divalent en C₁-C₁₈, par exemple en C₁-C₈ ;
Q⁻ est un ion halogénure, notamment chlorure ;
r représente une valeur statistique moyenne de 2 à 20 et en particulier de 2 à 8 ;
s représente une valeur statistique moyenne de 20 à 200 et en particulier de 20 à 50.

De tels polymères sont décrits plus particulièrement dans le brevet US 4 185 087.

(b) les composés de formule : NH-[(CH₂)₃-Si[OSi(CH₃)₃]]₃
correspondant à la dénomination CTFA "aminobispropyldiméthicone".

Un polymère entrant dans cette classe est le polymère commercialisé par la Société Union Carbide sous la dénomination "Ucar Silicone ALE 56.

Lorsque ces polymères siliconés sont mis en oeuvre, une forme de réalisation particulièrement intéressante est leur utilisation conjointe avec des agents de surface cationiques et/ou non ioniques. On peut utiliser par exemple le produit commercialisé sous la dénomination "Emulsion Cationique DC 929" par la Société DOW CORNING qui comprend, outre l'amodiméthicone, un agent de surface cationique comprenant un mélange de produits répondant à la formule (VIII) : 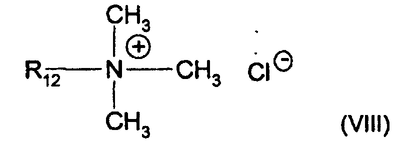 dans lequel R₁₂ désigne des radicaux alcényle et/ou alcoyle ayant de 14 à 22 atomes de carbone, dérivés des acides gras du suif,
en association avec un agent de surface non ionique de formule :

C₉H₁₉-C₆H₄-(OC₂H₄)₁₀-OH

connu sous la dénomination "Nonoxynol 10".

Un autre produit commercial utilisable selon l'invention est le produit commercialisé sous la dénomination "Dow Corning Q2 7224" par la Société Dow Corning comportant en association le triméthylsilylamodiméthicone de formule (IV), un agent de surface non ionique de formule : C₈H₁₇-C₆H₄-(OCH₂CH₂)ₙ-OH où n = 40 dénommé encore octoxynol-40, un autre agent de surface non ionique de formule : C₁₂H₂₅-(OCH₂-CH₂)ₙ-OH où n = 6 encore dénommé isolaureth-6, et du glycol.

Les polymères du type polyamine, polyaminoamide, polyammonium quaternaire, utilisables conformément à la présente invention, pouvant être notamment mentionnés, sont ceux décrits dans les brevets français n°2 505 348 ou 2 542 997. Parmi ces polymères, on peut citer :

(1) Les copolymères vinylpyrrolidone-acrylate ou -méthacrylate de dialkylamino-alkyle quaternisés ou non, tels que les produits commercialisés sous la dénomination "Gafquat®" par la Société ISP, comme par exemple Gafquat 734, 755 ou HS100 ou bien le produit dénommé "Copolymère 937". Ces polymères sont décrits en détail dans les brevets français 2 077 143 et 2 393 573.

(2) Les dérivés d'éthers de cellulose, notamment des hydroxyalkyl(C1-C4) cellulose, comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde (notamment épichlorhydrine) substitué par un groupement triméthylammonium.

(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyL triméthyl ammonium, de méthacrylmidopropyl triméthyl ammonium ou de diméthyldiallylammonium.

Les produits commercialisés répondant à cette définition sont plus particulièrement les produits commercialisés sous la dénomination "Celquat L 200" et "Celquat H 100" par la Société National Starch.

(4) Les polysaccharides cationiques décrits plus particulièrement dans les brevets US 3 589 578 et 4 031 307 et plus particulièrement le produit commercialisé sous la dénomination "Jaguar C.13 S" commercialisé par la Société MEYHALL.

(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.

(6) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quatemisées. De tels polymères sont notamment décrits dans les brevets français 2.252.840 et 2.368.508.

(7) Les dérivés de polyaminoamides résultant de la condensation de polyalcoylènes polyamines avec des acides polycarboxyliques, suivie d'une alcoylation par des agents bifonctionnels. On peut citer par exemple les polymères acide adipique-diacoylaminohydroxyalcoyldialcoylène triamine dans lesquels le radical alcoyle comporte de 1 à 4 atomes de carbone et désigne de préférence méthyle, éthyle, propyle. De tels polymères sont notamment décrits dans le brevet français 1.583.363.

Parmi ces dérivés, on peut citer plus particulièrement les polymères acide adipique/diméthylaminohydroxypropyl/diéthylène triamine commercialisés sous la dénomination "Cartaretine F, F4 ou F8" par la société Sandoz.

(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5:1 et 1,8:1. De tels polymères sont notamment décrits dans les brevets américains 3.227.615 et 2.961.347.

Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.

(9) les cyclopolymères de méthyl diallyl amine ou de diallyl diméthyl ammonium tels que les homopolymères ou les copolymères comportant comme constituant principal de la chaîne des motifs répondant aux formules (IX) ou (IX') : 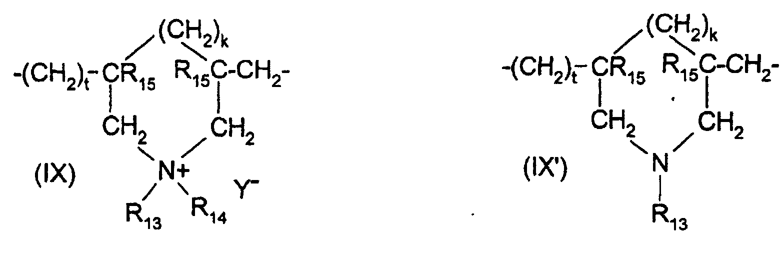 formules dans lesquelles k et t sont égaux à 0 ou 1, la somme k + t étant égale à 1 ; R₁₅ désigne un atome d'hydrogène ou un radical méthyle ; R₁₃ et R₁₄, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ou R₁₃ et R₁₄ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.

On peut citer par exemple l'homopolymère de chlorure de diallyldiméthylammonium commercialisé sous la dénomination "MERQUAT 100" par la société MERCK et les copolymères de chlorure de diallyldiméthylammonium et d'acrylamide commercialisés sous la dénomination "MERQUAT 550".

(10) le polymère de diammonium quaternaire contenant des motifs récurants répondant à la formule (X) : 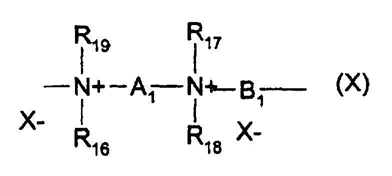 formule (X) dans laquelle :
R₁₆, R₁₇, R₁₈ et R₁₉, identiques ou différents, représentent des radicaux aliphatiques, alicycliques, ou arylaliphatiques contenant de 1 à 20 atomes de carbone ou des radicaux hydroxyalkylaliphatiques inférieurs, ou R₁₆, R₁₇, R₁₈ et R₁₉, ensemble ou séparément, constituent avec les atomes d'azote auxquels ils sont rattachés des hétérocycles contenant éventuellement un second hétéroatome autre que l'azote ou bien R₁₆, R₁₇, R₁₈ et R_{19,} représentent un radical alkyle en C₁-C₆ linéaire ou ramifié substitué par un groupement nitrile, ester, acyle, amide ou -CO-O-R₂₀-D ou -CO-NH-R₂₀-D où R₂₀ est un alkylène et D un groupement ammonium quaternaire ;
A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique ;
A1, R₁₆ et R₁₈ peuvent former avec les deux atomes d'azote auxquels ils sont rattachés un cycle pipérazinique ; en outre si A₁ désigne un radical alkylène ou hydroxyalkylène, linéaire ou ramifié, saturé ou insaturé, B₁ peut également désigner un groupement

   (CH2)ₙ-CO-D-OC-(CH2)ₙ- .
dans lequel n désigne un entier allant de 1 à 6, D désigne :
a) un reste de glycol de formule : -O-Z-O-, où Z désigne un radical hydrocarboné linéaire ou ramifié ou un groupement répondant à l'une des formules suivantes :

   - (CH₂-CH₂-O)x-CH₂-CH₂-

   - [CH₂-CH(CH₃)-O]_{y}-CH₂-CH(CH₃)-

   où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent

   -CH₂-CH₂-S-S-CH₂-CH₂- ;
d) un groupement uréylène de formule : -NH-CO-NH-.

De préférence, X⁻ est un anion tel que le chlorure ou le bromure.

Ces polymères ont une masse moléculaire moyenne en nombre généralement comprise entre 1000 et 100000.

Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

(11) les polymères de polyammonium quaternaires constitués de motifs de formule (XI) : 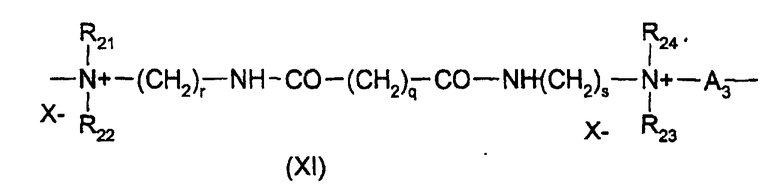 formule dans laquelle :
R₂₁, R₂₂, R₂₃ et R₂₄, identiques ou différents, représentent un atome d'hydrogène ou un radical méthyle, éthyle, propyle, β-hydroxyéthyle, β-hydroxypropyle ou - CH₂CH₂(OCH₂CH₂)ₚOH,
où p est égal à 0 ou à un nombre entier compris entre 1 et 6, sous réserve que R₂₁, R₂₂, R₂₃ et R₂₄ ne représentent pas simultanément un atome d'hydrogène,
r et s, identiques ou différents, sont des nombres entiers compris entre 1 et 6,
q est égal à 0 ou à un nombre entier compris entre 1 et 34,
X désigne un atome d'halogène,
A₃ désigne un radical d'un dihalogénure ou représente de préférence -CH₂-CH₂-O-CH₂-CH₂-.

De tels composés sont notamment décrits dans la demande de. brevet EP-A-122 324.

On peut par exemple citer parmi ceux-ci, les produits "Mirapol® A 15", "Mirapol® AD1", "Mirapol® AZ1" et "Mirapol® 175" commercialisés par la société Miranol.

(12) les homopolymères ou copolymères dérivés des acides acrylique ou méthacrylique et comportant des motifs de formules (XII), (XIII), (XIV) suivants : 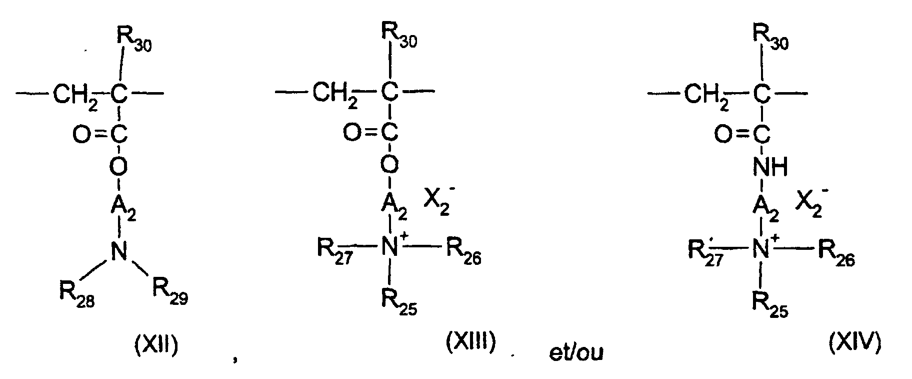 dans lesquels les groupements R₃₀ désignent indépendamment H ou CH₃,
les groupements A₂ désignent indépendamment un groupe alcoyle linéaire ou ramifié de 1 à 6 atomes de carbone ou un groupe hydroxyalcoyle de 1 à 4 atomes de carbone,
les groupements R₂₅, R₂₆, R₂₇, identiques ou différents, désignant indépendamment un groupe alcoyle de 1 à 18 atomes de carbone ou un radical benzyle,
les groupements R₂₈ et R₂₉ représentent un atome d'hydrogène ou un groupement alcoyle de 1 à 6 atomes de carbone,
X₂⁻ désigne un anion, par exemple méthosulfate ou halogénure, tel que chlorure ou bromure.

Le ou les comonomères utilisables dans la préparation des copolymères correspondants appartiennent à la famille des acrylamides, méthacrylamides, diacétone acrylamides, acrylamides et méthacrylamides substitués à l'azote par des alcoyle inférieurs, des esters d'alcoyles, des acides acrylique ou méthacrylique, la vinylpyrrolidone ou des esters vinyliques.

(13) Les polymères quaternaires de vinylpyrrolidone et de vinylimidazole tels que par exemple les produits commercialisés sous les dénominations LUVIQUAT® FC 905, FC 550 et FC 370 par la société BASF.

(14) Les polyamines comme le Polyquart H commercialisé par HENKEL, référencé sous le nom de « POLYETHYLENEGLYCOL (15) TALLOW POLYAMINE » dans le dictionnaire CTFA.

(15) Les polymères réticulés de chlorure de méthacryloyloxyéthyl triméthyl ammonium tels que les polymères obtenus par homopolymérisation du diméthylaminoéthylméthacrylate quatemisé par le chlorure de méthyle, ou par copolymérisation de l'acrylamide avec le diméthylaminoéthylméthacrylate quatemisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE SC 92 » par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale. Cette dispersion est commercialisée sous le nom de « SALCARE® SC 95 » par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les cyclopolymères, en particulier les copolymères du chlorure de diméthyldiallylammonium et d'acrylamide ayant un poids moléculaire supérieur à 500 000, commercialisés sous les dénominations « MERQUAT® 550 » et « MERQUAT® S » par la Société MERCK, les polysaccharides cationiques et plus particulièrement le polymère commercialisé sous la dénomination « JAGUAR® C13S » par la Société MEYHALL, et les polyaminoamides de la famille (6) décrits ci-dessus.

Selon l'invention, on peut également utiliser des polymères cationiques sous forme de latex ou de pseudolatex, c'est à dire sous forme d'une dispersion de particules de polymères insolubles.

Selon l'invention, le ou les polymères cationiques peuvent être présents en une teneur allant de 0,01% à 20 % en poids, de préférence de 0,01 % à 15 % en poids, et encore plus préférentiellement de 0,1 % à 5 % en poids, du poids total de la composition.

Le rapport charge cationique du(es) polymère(s) cationique(s) / charge anionique du(es) polymère(s) anionique(s) exprimée en meq./g est généralement compris entre 0,25 et 5, de préférence entre 0,5 et 2 et encore plus préférentiellement entre 0,75 et 1,25.

La charge cationique est le nombre d'atome d'amine quaternaire, tertiaire, secondaire ou primaire par gramme de polymère.

Avantageusement, le polymère cationique peut être une hydroxyalkyl(C1-C4)cellulose comportant des groupements ammonium quaternaires, notamment une hydroxyéthylcellulose réticulée à l'épichlorhydrine quatemisée par la triméthylamine ; le polymère anbionique peut être un polyméthacrylate de sodium.

La composition selon l'invention peut comprendre, en outre, au moins une cire. La cire peut être choisie parmi les cires d'origine animale, les cires d'origine végétale, les cires d'origine minérale, les cires synthétiques et les fractions diverses de cires d'origine naturelle. Les cires peuvent être présentes en une teneur allant de 2 % à 40 % en poids, par rapport au poids total de la composition, de préférence de 5 % à 30 % en poids, et mieux de 10 % à 25 % en poids.

Avantageusement, la cire peut être choisie parmi les cires (I) ayant un point de fusion allant de 70 °C à 110 °C. Ces cires ont notamment une pénétrabilité à l'aiguille allant de 1 à 7, 5. La pénétrabilité à l'aiguille des cires est déterminée se-Ion la norme française NF T 60-123 ou la norme américaine ASTM D 1321, à la température de 25 °C. Selon ces normes, la pénétrabilité à l'aiguille est la mesure de la profondeur, exprimée en dixièmes de millimètre, à laquelle une aiguille normalisée, pesant 2,5 g disposée dans un équipage mobile pesant 97,5 g et placée sur la cire à tester, pendant 5 secondes, pénètre dans la cire.

Les cires (I) peuvent par exemples être choisies notamment parmi la cire de son de riz, la cire de Carnauba, la cire d'Ouricuri, la cire de Candellila, les cires de Monatan, la cire de canne à sucre, certaines cires de polyéthylène qui répondent aux critères des cires (I).

Avantageusement, la composition selon l'invention peut comprendre une quantité de cires (I) allant de 0,1 % à 20 % en poids, par rapport au poids total de la composition, de préférence de 1 % à 10 % en poids.

Selon un mode de réalisation de la composition selon l'invention, la composition peut comprendre au moins une cire (la) ayant un point de fusion supérieur ou égal à 70 °C et inférieur à 83 °C et/ou une une cire (lb) ayant un point de fusion allant de 83 °C à 110 °C.

Comme cire (la), on peut par exemple citer la cire de son de riz ou la cire de Candelilla. Comme cire (Ib), on peut citer par exemple la cire de Carnauba, la cire d'Ouricuri, les cires de Montan. On utilise de préférence la cire de Carnauba.

Avantageusement, la composition selon l'invention peut comprendre un mélange de cires (I) contenant au moins une première cire (la) et au moins une deuxième cire (Ib) telles que définies précédemment.

Ledit mélange de cires (I) peut comprendre de 5 % à 50 % en poids de cire (Ia), par rapport au poids total dudit mélange de cires (I), et de 50 % à 95 % en poids de cire (Ib).

La composition peut comprendre, en outre, au moins une cire (II), dite cire molle, ayant un point de fusion supérieur ou égal à 45 °C et inférieur à 70 °C. La cire (II) peut avantageusement avoir une pénétrabilité à l'aiguille supérieure à 7,5, et de préférence inférieure ou égale à 217, mesurée selon les conditions définies précédemment pour les cires (I). Cette cire (II) permet notamment d'assouplir le revêtement déposé sur les cils.

Ces cires (II) peuvent être notamment choisies parmi la cire d'abeilles, les cires de lanoline, les cires de paraffine, les cires de cérasine, les cires microcristallines, les ozokérites, les spermaceti, certaines cires de polyéthylène de poids moléculaire tel qu'elles répondent aux critères des cires II, les huiles végétales hydrogénées.

Parmi les huiles végétales hydrogénées, on peut citer les cires de jojoba hydrogénées et les huiles hydrogénées qui sont obtenues par hydrogénation catalytique de corps gras composés de chaîne grasse linéaire ou non en C₈-C₃₂ et qui ont les qualités correspondant à la définition des cires. On peut citer notamment l'huile de tournesol hydrogénée, l'huile de ricin hydrogénée, l'huile de coton hydrogénée, l'huile de coprah hydrogénée et la lanoline hydrogénée.

Avantageusement, la cire (I) et la cire (II) peuvent être présentes dans la composition selon un rapport pondéral cire (I) / cire (II) pouvant aller de 0,2 à 1, et de préférence de 0,4 à 0,7.

La composition peut contenir, en outre, au moins un polymère filmogène non-ionique, différent du copolymère d'acrylate d'éthyle et de méthacrylate de méthyle défini précédemment, en une teneur pouvant aller de 0 % à 15 % en poids (notamment 0,1 % à 15 % en poids), par rapport au poids total de la composition, et de préférence de 0,1 % à 10 % en poids.

Comme polymère filmogène non-ionique, on peut par exemple citer les
- les polymères de cellulose tels que l'hydroxyéthylcellulose, l'hydroxypropyl-cellulose, l'hydroxypropyl éthylcellulose, l'éthylhydroxyéthyl-cellulose ;
- les polymères ou copolymères d'esters acryliques, tels que les polyacrylates ou les polyméthacrylates ;
- les polymères vinyliques, comme les polyvinylpyrrolidones, les copolymères de vinylpyrrolidone et d'acétate de vinyle ; l'alcool polyvinylique ;
- les polyesters, les polyamides, et les résines époxyesters ;
- les polymères polyuréthanes, les polyuréthanes-polyvinylpirrolidones, les polyester-polyuréthanes, les polyéther-polyuréthanes, les polyurées, les polyurée/polyuréthanes,
- les polymères d'origine naturelle, éventuellement modifiés, tels que les gommes arabiques, la gomme de guar, les dérivés du xanthane, la gomme de karaya ;
- et leurs mélanges.

La composition peut comprendre avantageusement une silicone polyoxyalkylénée, notamment choisie parmi les silicones comprenant une chaîne polyoxyalkylénée pendante ou terminale, ou bien encore un bloc polyoxyalkyléné. Comme chaîne ou bloc polyoxyalkylénée, on peut notamment citer les chaînes ou blocs polyoxyéthylénés ou polyoxypropylénés.

La silicone polyoxyalkylénée peut être notamment choisie parmi les composés de formule générale (XV) 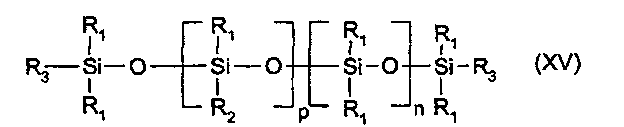 formule dans laquelle
- R₁, identiques ou différents, représentent un atome d'hydrogène, un radical alkyle linéaire ou ramifié en C₁-C₃₀ ou un radical phényle,
- R₂, identique ou différent, représente R₁ ou A = -(CₓH₂ₓ)-(OC₂H₄)ₐ-(OC₃H₆)_{b}-OR₄,
- R₃, identiques ou différents, désignent R, ou A, avec R₂ différent de R₃ quand R₂ = A ou R₃ = A,
- R₄, identiques ou différents, sont choisis parmi un atome d'hydrogène, un radical alkyle, linéaire ou ramifié, ayant de 1 à 12 atomes de carbone, un radical acyle, linéaire ou ramifié ayant de 2 à 6 atomes de carbone,
- n varie de 0 à 1000,
- p varie de 1 à 50,
- a varie de 0 à 50,
- b varie de 0 à 50,
- a + b est supérieur ou égal à 1,
- x varie de 1 à 5,
- le poids moléculaire moyen en nombre étant supérieur ou égal à 900 et de préférence compris entre 2000 et 75000,
- et leurs mélanges.

De préférence, la silicone polyoxyalkylénée peut être un (di)méthicone copolyol.

De façon préférentielle, on utilise les silicones polyoxyalkylénées de formule générale (XV) qui répondent à au moins une des, et de préférence à toutes les, conditions suivantes :
- R₁ désigne le radical méthyle,
- R₂ = A,
- R₃ = R₁,
- R₄ représente un atome d'hydrogène, un radical méthyle ou un radical acétyle, et de préférence hydrogène,
- p varie de 8 à 20,
- a est compris entre 5 et 40 et de préférence entre 15 et 30,
- b est compris entre 5 et 40 et de préférence entre 15 et 30,
- x est égal à 2 ou 3,
- n varie de 20 à 600, de préférence de 50 à 500 et encore plus particulièrement de 100 à 300,
- et leurs mélanges.
De telles silicones sont par exemple décrites dans le brevet US-4,311,695 qui est inclus à titre de référence.

Des silicones polyoxyalkylénées ont en particulier été présentés par la société DOW CORNING lors du 17ème congrès international de l'I.F.S.C.C. d'octobre 1992 et rapportés dans l'article "Water-soluble dimethicone copolyol waxes for personal care industry" de Linda Madore et al., pages 1 à 3. On peut également utiliser celles décrites dans la demande EP-A-331833 dont le contenu est inclus dans la présente demande à titre de référence.
Ces silicones polyoxyalkylénées sont des polydiméthylsiloxanes (PDMS) comportant une ou plusieurs fonctions éthers, solubles dans l'eau (oxyalkylène, notamment oxyéthylène et/ou oxypropylène).
De telles silicones polyoxyalkylénées sont notamment vendues par la société GOLDSCHMIDT sous la dénomination ABIL B8851, ABIL B88183, ABIL WE09, ABIL EM90, ABIL EM97. On peut citer aussi les composés KF 351 à 354 et KF 615 A vendus par la société SHIN ETSU ou la DMC 6038 de la société WACKER.
Les dérivés de diméthicones copolyols utilisables peuvent être en particulier les diméthicones copolyols à groupement phosphate, sulfate, chlorure de myristamide propyldiméthylammonium, stéarate, amine, glycomodifié, etc. On peut utiliser comme dérivés de diméthicones copolyols notamment les composés vendus par la société SILTECH sous la dénomination Silphos A100, Siltech amine 65, Silwax WDIS, myristamido silicone quat, ou par la société PHOENIX sous la dénomination Pecosil PS 100.

On peut également utiliser les dérivés vendus par la société WACKER sous la dénomination BELSIL DMC6031, ou par la société DOW CORNING sous la dénomination 2501 cosmetic wax.

Avantageusement, la silicone polyoxyalkylénée peut être non ionique.

Les silicones polyoxyalkylénées les plus particulièrement préférées sont par exemple celles vendues par la société DOW CORNING sous la dénomination commerciale Q2-5220 et par la société RHONE POULENC sous la dénomination MIRASIL DMCO.

Lorsque le polymère anionique est une silicone, la silicone polyoxyalkylénée selon l'invention sera différente dudit polymère anionique et par exemple une silicone non ionique. A titre d'exemple particulier, le polymère anionique peut être une diméthicone copolyol à groupement phosphate comme le Pecosil PS100 et la silicone polyoxyéthylénée, une diméthicone copolyol non ionique comme l'ABIL EM 90 ou EM 97.

Lorsque la silicone polyoxyalkylénée comprend au moins un bloc polyoxyalkyléné, on peut utiliser des copolymères blocs linéaires polysiloxane-polyoxyalkylène, et notamment ceux répondant à la formule générale (XVI) suivante :

([Y(R₂SiO)ₐ' R'₂SiYO][(C_{n'}H_{2n'}O)_{b'}])_{c'} (XVI)

dans laquelle :
- R et R' identiques ou différents, représentent un radical hydrocarboné monovalent ne contenant pas d'insaturation aliphatique,
- n' est un nombre entier compris entre 2 et 4,
- a' est un nombre entier supérieur ou égal à 5,
- b' est un nombre entier supérieur ou égal à 4,
- c' est un nombre entier supérieur ou égal à 4,
- Y représente un groupe organique divalent qui est lié à l'atome de silicium adjacent par une liaison carbone-silicium et à un bloc polyoxyalkylène par un atome d'oxygène,
- le poids moléculaire moyen de chaque bloc siloxane est compris entre environ 400 et environ 10.000, celui de chaque bloc polyoxyalkylène étant compris entre environ 300 et environ 10.000,
- les blocs siloxane représentent de 10% environ à 90% environ en poids du copolymère bloc,
- le poids moléculaire moyen du copolymère bloc étant d'au moins 3.000,
- et leurs mélanges.

Les radicaux R et R' sont plus préférentiellement choisis dans le groupe comprenant les radicaux alkyle comme par exemple les radicaux méthyle, éthyle, propyle, butyle, pentyle, hexyle, octyle, décyle, dodécyle; les radicaux aryle comme par exemple phényle, naphtyle; les radicaux aralkyle comme par exemple benzyle, phényléthyle; les radicaux tolyle, xylyle et cyclohexyle.

Le radical divalent Y est de préférence -R"-, -R"-CO-, -R"-NHCO-,
- R"-NH-CO-NH-R"'-NHCO ou -R"-OCONH-R"'-NHCO-, où R" est un groupe alkylène divalent comme par exemple l'éthylène, le propylène ou le butylène et R"' est un groupe alkylène divalent ou un groupe arylène divalent comme -C₆H₄-, -C₆H₄-C₆H₄-, -C₆H₄-CH₂-C₆H₄- ou -C₆H₄-CH(CH₃)₂-C₆H₄-.
Encore plus préférentiellement Y représente un radical alkylène divalent, plus particulièrement le radical -CH₂-CH₂-CH₂-.

La préparation des copolymères blocs mis en oeuvre selon la présente invention est notamment décrite dans la demande européenne EP 0 492 657 A1.

Selon un mode de réalisation particulier de l'invention le copolymère bloc est choisi parmi les copolymères suivants :

[[(CH₃)₂SiO]₄₁(CH₃)₂SiCH₂CH(CH₃)CH₂-O(C₂H₄O)₁₈-

(C₃H₆O)₃₃CH₂CH(CH₃)CH₂]_{16.1}

[[(CH₃)₂SiO]₃₁(CH₃)₂SiCH₂CH(CH₃)CH₂-O(C₂H₄O)₂₀-

(C₃H₆O)₂₉CH₂CH(CH₃)CH₂]_{13.3}

[[(CH₃)₂SiO]₉(CH₃)₂SiCH₂CH(CH₃)CH₂-O(C₂H₄O)₂₀-

(C₃H₆O)₂₉CH₂CH(CH₃)CH₂]_{26.3}

[[(CH₃)₂SiO]₁₆(CH₃)₂SiCH₂CH(CH₃)CH₂-O(C₂H₄O)₁₈-

(C₃H₆O)₂₀CH₂CH(CH₃)CH₂]_{21.5}

[[(CH₃)₂SiO]₉(CH₃)₂SiCH₂CH(CH₃)CH₂-O(C₂H₄O)₅-CH₂CH(CH₃)CH₂]_{4.8}

Les valeurs décimales correspondent à des mélanges de composés de formule (XVI) et de valeur c' différent.

Les agents siliconés utilisés dans les compositions de l'invention peuvent être hydrosolubles ou liposolubles.

Dans la composition selon l'invention, la silicone polyoxyalkylénée peut être présente en une teneur allant de 0,01 à 5 % en poids, par rapport au poids total de la composition, et de préférence de 0,1 à 1,5 % en poids.

La composition peut comprendre de 10 à 30% de silicone polyoxyalkylénée en poids par rapport au poids total de polymère filmogène.

La composition selon l'invention peut comprendre de l'eau et se présenter sous la forme de dispersion cire-dans-eau, eau-dans cire, huile-dans-eau et eau-dans-huile. La teneur en eau dans la composition peut aller de 1 à 95 % en poids, par rapport au poids total de la composition, et mieux de 10 à 80 % en poids.

La composition selon l'invention peut comprendre en outre au moins une huile volatile. On entend par "huile volatile" une huile susceptible de s'évaporer à température ambiante d'un support sur lequel elle a été appliquée, autrement dit une huile ayant une tension de vapeur mesurable à température ambiante.

On peut notamment utiliser une ou plusieurs huiles volatiles à température ambiante et pression atmosphérique ayant par exemple une tension de vapeur, à pression et température ambiante > 0 mm de Hg (0 Pa) et en particulier allant de 10⁻³ à 300 mm de Hg (0,13 Pa à 40.000 Pa), à condition que la température d'ébullition soit supérieure à 30°C. Ces huiles volatiles sont favorables à l'obtention d'un film à propriétés "sans transfert" total et de bonne tenue. Ces huiles volatiles facilitent, en outre, l'application de la composition sur la peau, les muqueuses, les phanères. Ces huiles peuvent être des huiles hydrocarbonées, des huiles siliconées, des huiles fluorées, ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles préférées convenant pour la composition selon l'invention sont en particulier les huiles hydrocarbonées ayant de 8 à 16 atomes de carbones, et notamment les isoalcanes en C8-C16 (ou isoparaffines) et les esters ramifiés en C8-C16 comme l'isododécane (encore appelé 2,2,4,4,6-pentaméthylheptane), l'isodécane, l'isohexadécane, le néo pentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées.

Comme huiles volatiles, on peut aussi utiliser les silicones volatiles, comme par exemple les huiles de silicones cycliques et volatiles, notamment celles ayant une viscosité ≤ 8 centistokes (8 10⁻⁶ m²/s), telles que l'octaméthylcyclotétrasiloxane, le décaméthylcyclopentasiloxane, l'hexadécaméthylcyclohexasiloxane, les silicones linéaires volatiles telles que l'octaméthyltrisiloxane, l'heptaméthylhexyltrisiloxane, l'heptaméthyloctyltrisiloxane, le décaméthyltétrasiloxane, ou bien encore les huiles volatiles fluorées telles que le nonafluorométhoxybutane ou le perfluorométhylcyclopentane.

L'huile volatile peut être présente dans la composition selon l'invention en une teneur allant de 0 % à 80 % en poids (notamment de 1 % à 80 %), par rapport au poids total de la composition, de préférence de 0 % à 65 % en poids (notamment de 1 % à 65 %).

La composition peut également comprendre au moins une huile non volatile, et notamment choisie parmi les huiles hydrocarbonées et/ou siliconées et/ou fluorées non volatiles.
Comme huile hydrocarbonée non volatile, on peut notamment citer :
- les huiles hydrocarbonées d'origine animale telle que le perhydrosqualène ;
- les huiles hydrocarbonées d'origine végétale telles que les triglycérides liquides d'acides gras de 4 à 10 atomes de carbone comme les triglycérides des acides heptanoïques ou octanoïque, ou encore les huiles de tournesol, de pépins de raisin, de sésame, de maïs, d'abricot, de ricin, d'avocat, d'olive ou de germes de céréales de soja, l'huile d'amande douce, de palme, de colza, de coton, de noisette, de macadamia, de jojoba, les triglycérides des acides caprylique/caprique comme ceux vendus par la société Stéarineries Dubois ou ceux vendus sous les dénominations Miglyol 810, 812 et 818 par la société Dynamit Nobel, l'huile de beurre de karité ;
- les hydrocarbures linéaires ou ramifiés, d'origine minérale ou synthétique tels que la vaseline, les polydécènes, le polyisobutène hydrogéné tel que le parléam ;
- les esters et les éthers de synthèse comme les huiles de formule R₁COOR₂ dans laquelle R₁ représente le reste d'un acide gras supérieur comportant de 6 à 29 atomes de carbone et R₂ représente une chaîne hydrocarbonée contenant de 3 à 30 atomes de carbone, telles que l'huile de Purcellin, le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyldodécyle ; les esters de polyols comme le dioctanoate de propylène glycol, le diheptanoate de néopentylglycol, le diisonanoate de diéthylène glycol et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide myristique, l'acide palmitique, l'acide stéarique, l'acide béhénique, l'acide oléique, l'acide linoléique, l'acide linolénique
ou l'acide isostéarique ;
et leurs mélanges.

Les huiles de silicone non volatiles utilisables dans la composition selon l'invention peuvent être des huiles de faible viscosité telles que les polysiloxanes linéaires dont le degré de polymérisation est de préférence de 6 à 2000 environ. On peut citer, par exemple, les polydiméthylsiloxanes (PDMS) de viscosité supérieure à 10 mPa.s, les phényl diméthicones, les phényl triméthicones, les polyphénylméthylsiloxanes et leurs mélanges.

Les huiles fluorées utilisables dans l'invention sont notamment des huiles fluorosiliconées, des polyéthers fluorés, des silicones fluorées telles que décrit dans le document EP-A-847752.

Les huiles non volatiles peuvent être présentes dans la composition selon l'invention en une teneur allant de 0 % à 50 % en poids (notamment 0,1 à 50 %), par rapport au poids total de la composition, de préférence de 0 % à 20 % en poids (notamment 0,1 % à 20 %).

La composition selon l'invention peut contenir des agents tensioactifs émulsionnants présents notamment en une proportion allant de 2 à 30 % en poids par rapport au poids total de la composition, et mieux de 5 % à 15 %. Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition selon l'invention sont choisis :
- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de C₁-C₆ alkyl glucose polyoxyéthylénés, et leurs mélanges.
- parmi les tensioactifs anioniques : les acides gras en C16-C30 neutralisés par les amines, l'ammoniaque ou les sels alcalins, et leurs mélanges.

On utilise de préférence des tensioactifs permettant l'obtention d'émulsion huile-dans-eau ou cire-dans-eau.

La composition peut également comprendre au moins une matière colorante comme les composés pulvérulents, par exemple à raison de 0,01 à 25 % du poids total de la composition. Les composés pulvérulents peuvent être choisis parmi les pigments et/ou les nacres et/ou les charges habituellement utilisés dans les mascaras.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être choisies parmi celles bien connues de l'homme du métier et couramment utilisées dans les compositions cosmétiques. Comme charge, on peut notamment utiliser :
- le talc qui est un silicate de magnésium hydraté utilisé sous forme de particules généralement inférieures à 40 microns,
- les micas qui sont des aluminosilicates de compositions variées se présentant sous la forme d'écailles ayant des dimensions de 2 à 200 microns, de préférence de 5 à 70 microns et une épaisseur comprise entre 0,1 à 5 microns, de préférence de 0,2 à 3 microns, ces micas pouvant être d'origine naturelle telle que la muscovite la margarite, la roscoelithe, la lipidolithe, la biotite ou d'origine synthétique,
- l'amidon en particulier l'amidon de riz,
- le kaolin qui est un silicate d'aluminium hydraté qui se présente sous la forme de particules de forme isotrope ayant des dimensions généralement inférieures à 30 microns,
- les oxydes de zinc et de titane généralement utilisés sous la forme de particules ayant des dimensions ne dépassant pas quelques microns,
- le carbonate de calcium, le carbonate ou l'hydrocarbonate de magnésium,
- la cellulose microcristalline,
- la silice,
- les poudres de polymères synthétiques tels que le polyéthylène, les polyesters (l'isophtalate ou le téréphtalate de polyéthylène), les polyamides tels que ceux vendus sous la dénomination commerciale de "Nylon" ou de "Téflon" et les poudres de silicone.

La composition selon l'invention peut également contenir des ingrédients couramment utilisés en cosmétique, tels que les oligo-éléments, les adoucissants, les séquestrants, les parfums, les huiles, les silicones, les épaississants, les vitamines, les protéines, les céramides, les plastifiants, les agents de cohésion ainsi que les agents alcalinisants ou acidifiants habituellement utilisés dans le domaine cosmétique, les émollients, les conservateurs.

Bien entendu, l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

La composition selon l'invention peut être préparée selon les méthodes usuelles des domaines considérés.

L'invention est illustrée plus en détail dans les exemples suivants.

### Exemple 1 :

On a préparé un mascara ayant la composition suivante :
- Cire de carnauba 7 g
- Cire d'abeille 8 g
- Cire de son de riz 7 g
- Cire de candelilla 2,5 g
- Amino-2 méthyl-2 propanediol-1,3 0,2 g
- Triéthanolamine 2,4 g
- Acide stéarique 5,4 g
- Polymères non-ioniques hydrosolubles 1,72 g
- Copolymère acrylate d'éthyle/méthacrylate de méthyle (80/20) en dispersion aqueuse à 50 % MA (DAITOSOL 5000 AD de SAITO) 0,75 g MA
- Diméthicone copolyol (Q2-5220 de DOW-CORNING) 0,2 g
- Polyméthacrylate de sodium (Darvan 7 de la société VANDERBILT) 0,25 g MA
- Hydroxyéthylcellulose réticulée par l'épichlorhydrine quatemisée par la triméthylamine (JR 400 de la société UNION CARBIDE) 0,1 g
- Pigments 6 g
- Conservateurs qs
- Eau qsp 100 g

Les critères de maquillage de la composition ont été évalués par un panel de 19 femmes.

La majorité du panel a jugé que la composition adhère bien sur les cils et s'appliquait facilement. La composition permet d'obtenir un maquillage rapidement : les cils sont bien allongés et recourbés. Le maquillage obtenu est confortable et présente une bonne tenue dans le temps (moins d'effritement qu'un mascara de l'état de la technique) ; les cils sont également bien séparés ; le mascara se démaquille aussi facilement.

## Revendications

1. Composition cosmétique de revêtement des fibres kératiniques comprenant au moins un polymère filmogène comprenant au moins un polymère cationique et au moins un polymère anionique, avantageusement non réticulé, **caractérisée par le fait qu'**elle comprend une dispersion aqueuse de polymère filmogène non ionique qui est un copolymère d'acrylate d'éthyle et de méthacrylate de méthyle.

2. Composition selon la revendication précédente, **caractérisée par le fait que** le copolymère d'acrylate d'éthyle et de méthacrylate de méthyle est présent en une teneur en matières sèches allant de 0,1 % à 15 % en poids, par rapport au poids total de la composition, et mieux de 0,5 % à 8 % en poids.

3. Composition selon l'une quelconque des revendications précédentes, **caractérisée par le fait que** le polymère anionique est choisi parmi :
- les polymères comportant des motifs carboxyliques dérivant de monomères mono ou diacides carboxyliques insaturés de formule (I) : 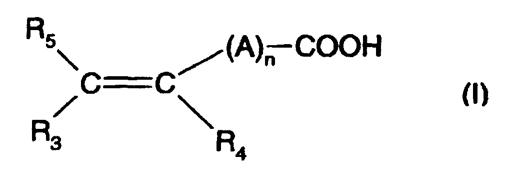
dans laquelle n est un nombre entier de 0 à 10, A désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₅ désigne un atome d'hydrogène, un groupement phényle ou benzyle, R₃ désigne un atome d'hydrogène, un groupement alkyle inférieur ou carboxyle, R₄ désigne un atome d'hydrogène, un groupement alkyle inférieur, un groupement -CH₂-COOH, phényle ou benzyle,
- les polymères comprenant des motifs dérivant d'acide sulfonique tels que des motifs vinylsulfonique, styrènesulfonique, acrylamido alkylsulfonique, et les polyesters sulfoniques,
- et leurs mélanges.

4. Composition selon l'une quelconque des revendications précédentes, **caractérisée en ce que** le polymère anionique est choisi parmi :
A) les homo- ou copolymères d'acide acrylique ou méthacrylique ou leurs sels, les copolymères d'acide acrylique et d'acrylamide et leurs sels, les sels de sodium d'acides polyhydroxycarboxyliques ;
B) les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol; les copolymères de ce type comportant dans leur chaîne un motif acrylamide éventuellement N-alkylé et/ou hydroxyalkylé, les copolymères d'acide acrylique et de méthacrylate d'alkyle en C₁-C₄ et les terpolymères de vinylpyrrolidone, d'acide acrylique et de méthacrylate d'alkyle en C₁-C₂₀ ;
C) les copolymères dérivés d'acide crotonique tels que ceux comportant dans leur chaîne des motif acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés ;
D) les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; les copolymères d'anhydrides maléique, citraconique, itaconique et d'un ester allylique ou méthallylique comportant éventuellement un groupement acrylamide, méthacrytamide, une α-oléfine, des esters acryliques ou méthacryliques, des acides acrylique ou méthacrylique ou la vinylpyrrolidone dans leur chaîne, les fonctions anhydrides sont monoestérifiées ou monoamidifiées ;
E) les polyacrylamides comportant des groupements carboxylates,
F) l'acide désoxyribonucléique ;
G) les copolymères d'au moins un diacide carboxylique, d'au moins un diol et d'au moins un monomère aromatique bifonctionnel portant un groupement -SO₃M avec M représentant un atome d'hydrogène, un ion ammonium NH₄⁺ ou un ion métallique ;
- et leurs mélanges.

5. Composition selon rune quelconque des revendications précédentes, **caractérisée en ce que** le polymère anionique est choisi parmi :
- les homopolymères d'acide acrylique ou méthacrylique ;
- les copolymères d'acide acrylique tels que le terpolymère acide acrylique/acrytate d'éthyle/N-tertiobutylacrylamide ;
- les copolymères dérivés d'acide crotonique tels que les terpolymères acétate de vinyle / tertio-butyl benzoate de vinyle / acide crotonique et les terpolymères acide crotonique/acétate de vinyle/néododécanoate de vinyle ;
- les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique mono estérifié.
- les copolymères d'acide méthacrylique et de méthacrylate de méthyle ;
- les copolymères d'acide méthacrylique et d'acrylate d'éthyle ;
- les terpolymères de vinylpyrrolidone/acide acrylique/méthacrylate de lauryle ;
- les copolymères acétate de vinyle/acide crotonique ;
- les terpolymères acétate de vinyle/acide crotonique/polyéthylèneglycol ;
- les sulfopolyesters obtenus par condensation de di-éthylèneglycol, de cyclohexane di-méthanol, d'acide isophtalique, d'acide sulfoisophtalique,
- et leurs mélanges.

6. Composition selon l'une quelconque des revendications précédentes, **caractérisée par le fait que** le polymère ànionique est choisi parmi les polymères anioniques de type siliconés greffés comprenant une portion polysiloxane et une portion constituée d'une chaîne organique non-siliconée, l'une des deux portions constituant la chaîne principale du polymère l'autre étant greffée sur la dite chaîne principale.

7. Composition selon la revendication 6, **caractérisée par le fait que** le polymère siliconé greffé est choisi parmi les polymères siliconés comportant dans leur structure le motif de formule (III) suivant : 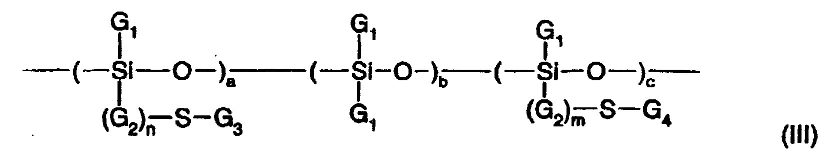 dans lequel les radicaux G₁, identiques ou différents, représentent l'hydrogène ou un radical alkyle en C₁-C₁₀ ou encore un radical phényle ; les radicaux G₂, identiques ou différents, représentent un groupe alkylène en C₁-C₁₀ ; G₃ représente un reste polymérique résultant de l'(homo)polymérisation d'au moins un monomère anionique à insaturation éthylénique ; G₄ représente un reste polymérique résultant de l'(homo)polymérisation d'au moins un monomère d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

8. Composition selon la revendication 7, **caractérisée par le fait que** le motif de formule (III) présente au moins l'une des caractéristiques suivantes :
- les radicaux G₁ désignent un radical alkyle en C₁-C₁₀ ;
- n est non nul, et les radicaux G₂ représentent un radical divalent en C₁-C₃ ;
- G₃ représente un radical polymérique résultant de l'(homo)polymérisation d'au moins un monomère du type acide carboxylique à insaturation éthylénique ;
- G₄ représente un radical polymérique résultant de l'(homo)polymérisation d'au moins un monomère du type (méth)acrylate d'alkyle en C₁-C₁₀ ;

9. Composition selon la revendication 7 ou 8, **caractérisée par le fait que** le motif de formule (III) présente simultanément les caractéristiques suivantes :
- les radicaux G₁ désignent un radical méthyle ;
- n est non nul, et les radicaux G₂ représentent un radical propylène ;
- G₃ représente un radical polymérique résultant de l'(homo)polymérisation d'au moins l'acide acrylique et/ou l'acide méthacrylique ;
- G₄ représente un radical polymérique résultant de l'(homo)polymérisation d'au moins le (méth)acrylate d'isobutyle ou de méthyle.

10. Composition selon l'une quelconque des revendications précédentes, **caractérisée par le fait que** le polymère cationique est choisi parmi les dérivés d'éther de cellulose quaternaires, les copotymères de cellulose avec un monomère hydrosoluble d'ammonium quaternaire, les cyclopolymères, les polysaccharides cationiques, les polymères cationiques siliconés, les copolymères vinylpyrrolidone-acrylate ou -méthacrylate de dialkylamino-alkyle quatemisés ou non, les polymères quaternaires de vinylpyrrolidone et de vinylimidazole, les polyaminoamides et leurs mélanges.

11. Composition selon l'une quelconque des revendications précédentes, **caractérisée par le fait que** le polymère anionique est un polyméthacrylate de sodium.

12. Composition selon l'une quelconque des revendications précédentes, **caractérisée par le fait que** le polymère cationique est une hydroxyalkyl(C1-C4)cellulose comportant des groupements ammonium quaternaires.

13. Composition cosmétique de revêtement des fibres kératiniques comprenant au moins un polymère filmogène comprenant un polymère cationique qui est une hydroxyalkyl(C1-C4)cellulose comportant des groupements ammonium quaternaires et un polymère anionique qui est un polyméthacrylate de sodium, **caractérisée par le fait qu'**elle comprend une dispersion aqueuse de polymère filmogène non-ionique qui est un copolymère d'acrylate d'éthyle et de méthacrylate de méthyle.

14. Composition selon la revendication 13, **caractérisée par le fait que** le polymère cationique est une hydroxyéthylcellulose réticulée à l'épichlorhydrine quaternisée par la triméthylamine.

15. Composition selon la revendication 13 ou 14, **caractérisée par le fait que** le copolymère d'acrylate d'éthyle et de méthacrylate de méthyle est présent en une teneur en matières sèches allant de 0,1 % à 15 % en poids, par rapport au poids total de la composition, et mieux de 0,5 % à 8 % en poids.

16. Composition selon l'une quelconque des revendications précédentes, **caractérisée par le fait que** le polymère cationique est présent en une teneur allant de 0,01 % à 20 % en poids, de préférence de 0,01 % à 15 % en poids, et encore plus préférentiellement de 0,1 % à 5 % en poids, du poids total de la composition.

17. Composition selon l'une quelconque des revendications précédentes, **caractérisée par le fait que** le polymère anionique est utilisé en une quantité allant de 0,01 à 20% en poids du poids total de la composition, de préférence de 0,05 à 15% en poids et encore plus préférentiellement 0,1 % à 7 % en poids.

18. Composition selon l'une quelconque des revendications précédentes, **caractérisée par le fait qu'**elle comprend, en outre, une cire.

19. Composition selon la revendication 18, **caractérisée par le fait que** la cire est présente en une teneur allant de 2 % à 40 % en poids, par rapport au poids total de la composition, de préférence de 5 % à 30 % en poids, et mieux de 10 % à 25 % en poids.

20. Composition selon l'une des revendications 18 ou 19, **caractérisée par le fait que** la cire comprend au moins une cire (I) ayant un point de fusion allant de 70 °C à 110 °C.

21. Composition selon la revendication 20, **caractérisée par le fait que** la cire (1) est présente en une teneur allant de 0,1 % à 20 % en poids, par rapport au poids total de la composition.

22. Composition selon l'une quelconque des revendications précédentes 18 à 21, **caractérisée par le fait que** la cire comprend au moins une cire (II) ayant un point de fusion supérieur ou égal à 45 °C et inférieur à 70 °C.

23. Composition selon la revendication 22, **caractérisée par le fait que** la cire (II) est présente selon un rapport pondéral cire (I)/cire (II) allant de 0,2 à 1.

24. Composition selon l'une quelconque des revendications précédentes, **caractérisée par le fait qu'**elle comprend, en outre, au moins une silicone polyoxyalkylénée.

25. Composition selon l'une quelconque des revendications précédentes, **caractérisée par le fait qu'**elle comprend au moins un diméthicone copolyol.

26. Composition selon l'une quelconque des revendications précédentes, **caractérisée par le fait qu'**elle comprend, en outre, au moins un polymère filmogène non-ionique différent du copolymère d'acrylate d'éthyle et de méthacrylate de méthyle défini selon l'une des revendications 1 ou 13.

27. Composition selon l'une quelconque des revendications précédentes, **caractérisée par le fait qu'**elle comprend, en outre, de l'eau en une teneur allant de 1 % à 95 % en poids, par rapport au poids total de la composition, et mieux de 10 à 80 % en poids.

28. Composition selon l'une quelconque des revendications précédentes, **caractérisé en ce qu'**elle est sous forme d'une émulsion cire-dans-eau, eau-dans-cire, huile-dans-eau, eau-dans-huile.

29. Composition selon l'une quelconque des revendications précédentes, **caractérisée par le fait qu'**elle comprend, en outre, au moins une huile volatile.

30. Composition selon l'une quelconque des revendications précédentes, **caractérisée par le fait qu'**elle comprend, en outre, au moins un tensioactif émulsionnant.

31. Composition selon la revendication 30, **caractérisée par le fait que** le tensioactif émulsionnant est présent en une teneur allant de 2 % à 30 % en poids, par rapport au poids total de la composition.

32. Composition selon l'une quelconque des revendications précédentes, **caractérisée par le fait qu'**elle comprend au moins un additif choisi dans le groupe formé par les vitamines, les oligo-éléments, les adoucissants, les séquestrants, les parfums, les huiles, les épaississants, les protéines, les céramides, les plastifiants, les agents de cohésion, les agents alcalinisants ou acidifiants, les charges, les pigments, les émollients, les conservateurs, et leurs mélanges.

33. Composition selon l'une quelconque des revendications précédentes, **caractérisée par le fait que** la composition est une composition de maquillage, une base de maquillage, une composition à appliquer sur un maquillage, une composition de traitement cosmétique des fibres kératiniques.

34. Procédé de revêtement des fibres kératiniques, notamment des cils, **caractérisé par le fait que** l'on applique sur les fibres kératiniques une composition selon l'une quelconque des revendications 1 à 33.

35. Utilisation d'une composition telle que définie selon l'une quelconque des revendications 1 à 33 pour recourber les cils et /ou allonger et/ou gainer les cils et/ou pour obtenir un maquillage rapide et/ou facile à appliquer et/ou de bonne tenue.

## Patentansprüche

1. Kosmetische Zusammensetzung zum Umhüllen von Keratinfasern, die mindestens ein filmbildendes Polymer enthält, das mindestens ein kationisches Polymer und mindestens ein anionisches Polymer umfasst, welches vorteilhaft nicht vernetzt ist, **dadurch gekennzeichnet, dass** sie eine wässrige Dispersion eines nichtionischen filmbildenden Polymers enthält, bei dem es sich um ein Ethylacrylat/Methylmethacrylat-Copolymer handelt.

2. Zusammensetzung nach dem vorhergehenden Anspruch, **dadurch gekennzeichnet, dass** das Ethylacrylat/Methylmethacrylat-Copolymer in einem Trockensubstanzgehalt von 0,1 bis 15 Gew.-%, bezogen auf das Gesamtgewicht der Zusammensetzung, und besser 0,5 bis 8 Gew.-% enthalten ist.

3. Zusammensetzung nach einem der vorhergehenden Ansprüche, **dadurch gekennzeichnet, dass** das anionische Polymer ausgewählt ist unter:
- Polymeren mit Carboxygruppen, die von ungesättigten Mono- oder Dicarbonsäuremonomeren der Formel (I) abgeleitet sind: 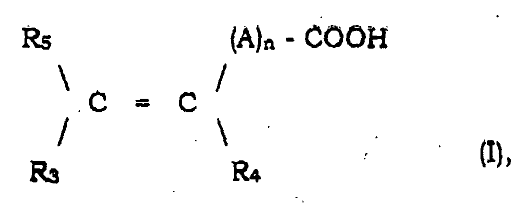
worin bedeuten:
n Null oder eine ganze Zahl von 1 bis 10, A eine Methylengruppe, die gegebenenfalls an das Kohlenstoffatom der ungesättigten Gruppe oder, falls n größer 1 ist, an die benachbarte Methylengruppe über ein Heteroatom, wie Sauerstoff oder Schwefel, gebunden ist, R₅ ein Wasserstoffatom, eine Phenylgruppe oder eine Benzylgruppe, R₃ ein Wasserstoffatom, eine niedere Alkylgruppe oder eine Carboxygruppe und R₄ ein Wasserstoffatom, eine niedere Alkylgruppe, eine Gruppe -CH₂-COOH, eine Phenylgruppe oder eine Benzylgruppe;
- Polymeren, die von einer Sulfonsäure abgeleitete Einheiten enthalten, wie beispielsweise Vinylsulfonsäure-Einheiten, Styrolsulfonsäure-Einheiten oder Acrylamidoalkylsulfonsäure-Einheiten, oder Sulfopolyestern;
- und deren Gemischen.

4. Zusammensetzung nach einem der vorhergehenden Ansprüche, **dadurch gekennzeichnet, dass** das anionische Polymer ausgewählt ist unter:
A) Homo- oder Copolymeren von Acrylsäure oder Methacrylsäure oder deren Salzen, Copolymeren von Acrylsäure und Acrylamid und deren Salzen und den Natriumsalzen von Polyhydroxycarbonsäuren,
B) Copolymeren von Acrylsäure oder Methacrylsäure mit einem monoethylenisch ungesättigten Monomer, wie Ethylen, Styrol, Vinylestern, Acrylsäure- oder Methacrylsäureestern, die gegebenenfalls auf ein Polyalkylenglykol, wie Polyethylenglykol, gepfropft sind; Copolymeren dieses Typs, die in ihrer Kette eine Acrylamideinheit aufweisen, die gegebenenfalls N-alkyliert und/oder hydroxyalkyliert ist; Copolymeren von Acrylsäure und C₁₋₄-Alkylmethacrylat und Terpolymeren von Vinylpyrrolidon, Acrylsäure und C₁₋₂₀-Alkylmethacrylat,
C) Copolymeren, die von Crotonsäure abgeleitet sind, wie Copolymeren, die in ihrer Kette Vinylacetat- oder Vinylpropionat-Einheiten und gegebenenfalls weitere Monomere aufweisen, wie Allylester oder Methallylester, Vinylether oder Vinylester einer gesättigten, geradkettigen oder verzweigten Carbonsäure mit langer Kohlenwasserstoffkette, wie Carbonsäuren mit mindestens 5 Kohlenstoffatomen, wobei diese Polymere gegebenenfalls gepfropft sein können,
D) Polymeren, die von Maleinsäure, Fumarsäure, Itaconsäure oder deren Anhydriden mit Vinylestern, Vinylethern, Vinylhalogeniden, Phenylvinylderivaten oder Acrylsäure und ihren Estern abgeleitet sind; Copolymeren von Maleinsäureanhydrid, Citraconsäureanhydrid oder Itaconsäureanhydrid mit einem Allylester oder einem Methallylester, die in ihrer Kette gegebenenfalls eine der folgenden Gruppen enthalten: Acrylamid, Methacrylamid, α-Olefin, Acrylester, Methacrylester, Acrylsäure, Methacrylsäure oder Vinylpyrrolidon, wobei die Anhydridgruppen einfach verestert sind oder als einfaches Amid vorliegen,
E) Polyacrylamiden mit Carboxylatgruppen,
F) Desoxyribonucleinsäure,
G) Copolymeren von mindestens einer Dicarbonsäure, mindestens einem Diol und mindestens einem bifunktionellen aromatischen Monomer mit einer Gruppe -SO₃M, worin M ein Wasserstoffatom, ein Ammoniumion NH₄⁺ oder ein Metallion bedeutet, und
den Gemischen dieser Verbindungen.

5. Zusammensetzung nach einem der vorhergehenden Ansprüche, **dadurch gekennzeichnet, dass** das anionische polymer ausgewählt ist unter:
- Homopolymeren von Acrylsäure oder Methacrylsäure,
- Copolymeren von Acrylsäure, wie dem Acrylsäure/Ethylacrylat/N*t*-Butylacrylamid-Terpolymer,
- Copolymeren, die von Crotonsäure abgeleitet sind, wie Vinylacetat/Vinyl-*t*-butylbenzoat/Crotonsäure-Terpolymeren und Crotonsäure /Vinylacetat/Vinylneododecanoat-Terpolymeren,
- Polymeren, die von Maleinsäure, Fumarsäure, Itaconsäure oder deren Anhydriden mit Vinylestern, Vinylethern, Vinylhalogeniden, Phenylvinylderivaten, Acrylsäure und ihren Estern abgeleitet sind, wie den Copolymeren von Methylvinylether und einfach verestertern Maleinsäureanhydrid,
- Copolymeren von Methacrylsäure und Methylmethacrylat,
- Copolymeren von Methacrylsäure und Ethylacrylat,
- Vinylpyrrolidon/Acrylsäure/Laurylmethacrylat-Terpolymeren,
- Vinylacetat/Crotonsäure-Copolymeren,
- Vinylacetat/Crotonsäure/Polyethylenglykol-Terpolymeren,
- Sulfopolyestern, die durch Kondensation von Diethylenglykol, Cyclohexandimethanol, Isophthalsäure und Sulfoisophthalsäure erhalten werden, und
- den Gemischen dieser Verbindungen.

6. Zusammensetzung nach einem der vorhergehenden Ansprüche, **dadurch gekennzeichnet, dass** das anionische Polymer unter anionischen Polymeren vom Typ der gepfropften Siliconpolymere ausgewählt ist, die einen Polysiloxan-Bestandteil und einen Bestandteil aufweisen, der aus einer nicht siliconhaltigen organischen Kette besteht, wobei ein Bestandteil die Hauptkette des Polymers bildet und der andere Bestandteil auf die Hauptkette gepfropft ist.

7. Zusammensetzung nach Anspruch 6, **dadurch gekennzeichnet, dass** das gepfropfte Siliconpolymer unter den Siliconpolymeren ausgewählt ist, die in ihrer Struktur die Einheit der folgenden Formel (III) aufweisen: 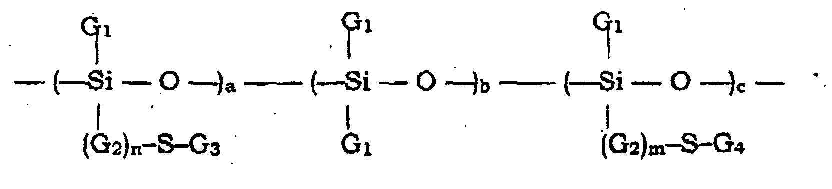 worin bedeuten:
die Gruppen G₁, die identisch oder voneinander verschieden sind, Wasserstoff oder eine Alkylgruppe mit 1 bis 10 Kohlenstoffatomen oder auch eine Phenylgruppe, die Gruppen G₂, die identisch oder voneinander verschieden sind, eine Alkylengruppe mit 1 bis 10 Kohlenstoffatomen, G₃ eine Polymergruppe, die das Ergebnis der (Homo)polymerisation mindestens eines anionischen Monomers mit ethylenischer Doppelbindung ist, G₄ eine Polymergruppe, die bei der (Homo)polymerisation mindestens eines hydrophoben Monomers mit ethylenischer Doppelbindung entsteht, m und n Null oder 1, a Null oder eine ganze Zahl von 1 bis 50, b eine ganze Zahl, die im Bereich von 10 bis 350 liegen kann, und c Null oder eine ganze Zahl von 1 bis 50,
mit der Maßgabe, dass einer der Parameter a und c von Null verschieden ist.

8. Zusammensetzung nach Anspruch 7, **dadurch gekennzeichnet, dass** die Einheit der Formel (111) mindestens eine der folgenden Eigenschaften aufweist:
- Die Gruppen G₁ bedeuten eine Alkylgruppe mit 1 bis 10 Kohlenstoffatomen,
- n ist nicht Null und die Gruppen G₂ bedeuten eine zweiwertige Gruppe mit 1 bis 3 Kohlenstoffatomen,
- G₃ bedeutet eine Polymergruppe, die das Ergebnis der (Homo)polymerisation mindestens eines Monomers vom Typ einer Carbonsäure mit ethylenischer Doppelbindung ist, und
- G₄ bedeutet eine Polymergruppe, die bei der (Homo)polymerisation mindestens eines Monomers vom Typ C₁₋₁₀-Alkyl(meth)acrylat gebildet wird.

9. Zusammensetzung nach Anspruch 7 oder 8, **dadurch gekennzeichnet, dass** die Einheit der Formel (III) gleichzeitig die folgenden Eigenschaften aufweist:
- Die Gruppen G₁ bedeuten eine Methylgruppe,
- n ist nicht Null und die Gruppen G₂ bedeuten eine Propylengruppe,
- G₃ bedeutet eine Polymergruppe, die durch (Homo)polymerisation von mindestens Acrylsäure und/oder Methacrylsäure entsteht, und
- G₄ bedeutet eine Polymergruppe, die das Ergebnis der (Homo)polymerisation von mindestens Isobutyl(meth)acrylat oder Methyl-(meth)acrylat ist.

10. Zusammensetzung nach einem der vorhergehenden Ansprüche, **dadurch gekennzeichnet, dass** das kationische Polymer ausgewählt ist unter quartären Celluloseetherderivaten, Cellulosecopolymeren mit einem wasserlöslichen quartären Ammoniummonomer, Cyclopolymeren, kationischen Polysacchariden, kationischen Siliconpolymeren, quaternisierten oder nicht quaternisierten Copolymeren von Vinylpyrrolidon und Dialkylaminoalkylacrylat oder Dialkylaminoalkylmethacrylat, quartären Polymeren von Vinylpyrrolidon und von Vinylimidazol, Polyaminoamiden und den Gemischen dieser Verbindungen.

11. Zusammensetzung nach einem der vorhergehenden Ansprüche, **dadurch gekennzeichnet, dass** das anionische Polymer ein Natriumpolymethacrylat ist.

12. Zusammensetzung nach einem der vorhergehenden Ansprüche, **dadurch gekennzeichnet, dass** das kationische Polymer eine C₁₋₄-Hydroxyalkylcellulose mit quartären Ammoniumgruppen ist.

13. Kosmetische Zusammensetzung zum Umhüllen von Keratinfasern, die mindestens ein filmbildendes Polymer enthält, das mindestens ein kationisches Polymer, bei dem es sich um eine C₁₋₄-Hydroxyalkylcellulose mit quartären Ammoniumgruppen handelt, und mindestens ein anionisches Polymer umfasst, bei dem es sich um ein Natriumpolymethacrylat handelt, **dadurch gekennzeichnet, dass** sie eine wässrige Dispersion eines nichtionischen filmbildenden Polymers enthält, bei dem es sich um ein Ethylacrylat/Methylmethacrylat-Copolymer handelt.

14. Zusammensetzung nach Anspruch 13, **dadurch gekennzeichnet, dass** das kationische Polymer eine Hydroxyethylcellulose ist, die mit mit Trimethylamin quaternisiertem Epichlorhydrin vernetzt ist.

15. Zusammensetzung nach Anspruch 13 oder 14, **dadurch gekennzeichnet, dass** das Ethylacrylat/Methylmethacrylat-Copolymer in einem Trockensubstanzgehalt von 0,1 bis 15 Gew.-%, bezogen auf das Gesamtgewicht der Zusammensetzung, und besser 0,5 bis 8 Gew.-% enthalten ist.

16. Zusammensetzung nach einem der vorhergehenden Ansprüche, **dadurch gekennzeichnet, dass** das kationische Polymer in einem Mengenanteil von 0,01 bis 20 Gew.-%, vorzugsweise von 0,01 bis 15 Gew.-% und noch bevorzugter von 0,1 bis 5 Gew.-% des Gesamtgewichts der Zusammensetzung vorliegt.

17. Zusammensetzung nach einem der vorhergehenden Ansprüche, **dadurch gekennzeichnet, dass** das anionische Polymer in einer Menge von 0,01 bis 20 Gew.-%, vorzugsweise 0,05 bis 15 Gew.-% und noch bevorzugter 0,1 bis 7 Gew.-% des Gesamtgewichts der Zusammensetzung verwendet wird.

18. Zusammensetzung nach einem der vorhergehenden Ansprüche, **dadurch gekennzeichnet, dass** sie ferner ein Wachs enthält.

19. Zusammensetzung nach Anspruch 18, **dadurch gekennzeichnet, dass** das Wachs in einem Mengenanteil von 2 bis 40 Gew.-%, bezogen auf das Gesamtgewicht der Zusammensetzung, vorzugsweise 5 bis 30 Gew.-% und besser 10 bis 25 Gew.-% vorliegt.

20. Zusammensetzung nach Anspruch 18 oder 19, **dadurch gekennzeichnet, dass** das Wachs mindestens ein Wachs (I) mit einem Schmelzpunkt von 70 bis 110 °C umfasst.

21. Zusammensetzung nach Anspruch 20, **dadurch gekennzeichnet, dass** das Wachs (I) in einer Menge von 0,1 bis 20 Gew.-%, bezogen auf das Gesamtgewicht der Zusammensetzung, vorliegt.

22. Zusammensetzung nach einem der Ansprüche 18 bis 21, **dadurch gekennzeichnet, dass** das Wachs mindestens ein Wachs (II) mit einem Schmelzpunkt von 45 °C oder darüber und unter 70 °C umfasst.

23. Zusammensetzung nach Anspruch 22, **dadurch gekennzeichnet, dass** das Wachs (II) in einem Gewichtsverhältnis von Wachs (I)/Wachs (II) von 0,2 bis 1 vorliegt.

24. Zusammensetzung nach einem der vorhergehenden Ansprüche, **dadurch gekennzeichnet, dass** sie ferner ein polyalkoxylierte Silicon enthält.

25. Zusammensetzung nach einem der vorhergehenden Ansprüche, **dadurch gekennzeichnet, dass** sie mindestens ein Dimethiconcopolyol enthält.

26. Zusammensetzung nach einem der vorhergehenden Ansprüche, **dadurch gekennzeichnet, dass** sie ferner mindestens ein nichtionisches filmbildendes Polymer enthält, das von dem in den Ansprüchen 1 oder 13 definierten Ethylacrylat/Methylmethacrylat-Copolymer verschieden ist.

27. Zusammensetzung nach einem der vorhergehenden Ansprüche, **dadurch gekennzeichnet, dass** sie außerdem Wasser in einem Mengenanteil von 1 bis 95 Gew.-% und besser von 10 bis 80 Gew.-%, bezogen auf das Gesamtgewicht der Zusammensetzung, enthält.

28. Zusammensetzung nach einem der vorhergehenden Ansprüche, **dadurch gekennzeichnet, dass** sie in Form einer Wachs-in-Wasser-Emulsion, einer Wasser-in-Wachs-Emulsion, einer Öl-in-Wasser-Emulsion oder einer Wasser-in-Öl-Emulsion vorliegt.

29. Zusammensetzung nach einem der vorhergehenden Ansprüche, **dadurch gekennzeichnet, dass** sie ferner mindestens ein flüchtiges Öl enthält.

30. Zusammensetzung nach einem der vorhergehenden Ansprüche, **dadurch gekennzeichnet, dass** sie mindestens einen emulgierenden grenzflächenaktiven Stoff enthält.

31. Zusammensetzung nach Anspruch 30, **dadurch gekennzeichnet, dass** der emulgierende grenzflächenaktive Stoff in einem Mengenanteil von 2 bis 30 Gew.-%, bezogen auf das Gesamtgewicht der Zusammensetzung, vorliegt.

32. Zusammensetzung nach einem der vorhergehende Ansprüche, **dadurch gekennzeichnet, dass** sie mindestens einen Zusatzstoff enthält, der unter den Vitaminen, Spurenelementen, reizlindernden Mitteln, Maskierungsmitteln, Parfums, Ölen, Verdickungsmitteln, Proteinen, Ceramiden, Weichmachern, Kohäsionsmitteln, Alkalisierungsmitteln, Ansäuerungsmitteln, Füllstoffen, Pigmenten, Emollientien, Konservierungsmitteln und deren Gemischen ausgewählt ist.

33. Zusammensetzung nach einem der vorhergehenden Ansprüche, **dadurch gekennzeichnet, dass** es sich bei der Zusammensetzung um eine Zusammensetzung zum Schminken, eine Grundmasse zum Schminken, eine Zusammensetzung zum Auftragen auf eine Schminke oder um eine Zusammensetzung zur kosmetischen Behandlung von Keratinfasern handelt.

34. Verfahren zum Umhüllen von Keratinfasern und insbesondere zum Umhüllen der Wimpern, **dadurch gekennzeichnet, dass** eine Zusammensetzung nach einem der Ansprüche 1 bis 33 auf die Keratinfasern aufgebracht wird.

35. Verwendung einer Zusammensetzung nach einem der Ansprüche 1 bis 33, um den Wimpern eine geschwungene Form zu geben und/oder sie zu verlängern und/oder sie zu umhüllen und/oder um eine rasch und/oder leicht aufzutragende und/oder gut haltbare Schminke zu erhalten.

## Claims

1. Cosmetic composition for coating keratin fibres, comprising at least one film-forming polymer comprising at least one cationic polymer and at least one anionic polymer, which is advantageously non-crosslinked, **characterized in that** it comprises an aqueous dispersion of nonionic film-forming polymer which is a copolymer of ethyl acrylate and of methyl methacrylate.

2. Composition according to either of the preceding claims, **characterized in that** the copolymer of ethyl acrylate and of methyl methacrylate is present in a solids content ranging from 0.1% to 15% by weight, relative to the total weight of the composition, and better still from 0.5% to 8% by weight.

3. Composition according to either of the preceding claims, **characterized in that** the anionic polymer is chosen from:
- polymers comprising carboxylic units derived from unsaturated mono- or dicarboxylic acid monomers of formula (I): 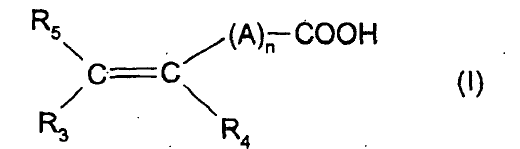 in which n is an integer from 0 to 10, A denotes a methylene group, optionally connected to the carbon atom of the unsaturated group or to the neighbouring methylene group when n is greater than 1 via a hetero atom such as oxygen or sulphur, R₅ denotes a hydrogen atom or a phenyl or benzyl group, R₃ denotes a hydrogen atom or a lower alkyl or carboxyl group, and R₄ denotes a hydrogen atom, a lower alkyl group or a -CH₂-COOH, phenyl or benzyl group,
- polymers comprising units derived from sulphonic acid, such as vinylsulphonic, styrenesulphonic and acrylamidoalkylsulphonic units and sulphonic polyesters,
- and mixtures thereof.

4. Composition according to any one of the preceding claims, **characterized in that** the anionic polymer is chosen from:
A) Homo- or copolymers of acrylic or methacrylic acid or salts thereof, copolymers of acrylic acid and of acrylamide and salts thereof, and sodium salts of polyhydroxycarboxylic acids;
B) Copolymers of acrylic or methacrylic acids with a monoethylenic monomer such as ethylene; styrene, vinyl esters, acrylic or methacrylic acid esters, which are optionally grafted onto a polyalkylene glycol such as polyethylene glycol; copolymers of this type whose chain comprises an optionally N-alkylated and/or hydroxyalkylated acrylamide unit, copolymers of acrylic acid and of C₁-C₄ alkyl methacrylate and terpolymers of vinylpyrrolidone, of acrylic acid and of C₁-C₂₀ alkyl methacrylate;
C) Copolymers derived from crotonic acid, such as those whose chain comprises vinyl acetate or propionate units and optionally other monomers such as allylic or methallylic esters, vinyl ether or vinyl ester of a saturated, linear or branched carboxylic acid containing a long hydrocarbon-based chain, such as those comprising at least 5 carbon atoms, it being possible for these polymers optionally to be grafted;
D) Polymers derived from maleic, fumaric or itaconic acids or anhydrides with vinyl esters, vinyl ethers, vinyl halides, phenylvinyl derivatives or acrylic acid and esters thereof; copolymers of maleic, citraconic or itaconic anhydrides and of an allylic or methallylic ester optionally comprising an acrylamide, methacrylamide, α-olefin, acrylic or methacrylic ester, acrylic or methacrylic acid or vinylpyrrolidone group in their chain, and the anhydride functions are monoesterified or monoamidated;
E) Polyacrylamides comprising carboxylate groups,
F) Deoxyribonucleic acid;
G) Copolymers of at least one dicarboxylic acid, of at least one diol and of at least one difunctional aromatic monomer bearing a group -SO₃M with M representing a hydrogen atom, an ammonium ion NH₄⁺ or a metal ion;
- and mixtures thereof.

5. Composition according to any one of the preceding claims, **characterized in that** the anionic polymer is chosen from:
- acrylic or methacrylic acid homopolymers;
- acrylic acid copolymers such as the acrylic acid/ethyl acrylate/N-tert-butylacrylamide terpolymer;
- copolymers derived from crotonic acid, such as vinyl acetate/vinyl tert-butylbenzoate/crotonic acid terpolymers and crotonic acid/vinyl acetate/vinyl neododecanoate terpolymers;
- polymers derived from maleic, fumaric or itaconic acids or anhydrides with vinyl esters, vinyl ethers, vinyl halides, phenylvinyl derivatives or acrylic acid and esters thereof, such as methyl vinyl ether/monoesterified maleic anhydride copolymers;
- copolymers of methacrylic acid and of methyl methacrylate;
- copolymers of methacrylic acid and of ethyl acrylate;
- terpolymers of vinylpyrrolidone/acrylic acid/lauryl methacrylate;
- vinyl acetate/crotonic acid copolymers;
- vinyl acetate/crotonic acid/polyethylene glycol terpolymers;
- sulphopolyesters obtained by condensation of diethylene glycol, cyclohexanedimethanol, isophthalic acid and sulphoisophthalic acid,
- and mixtures thereof.

6. Composition according to any one of the preceding claims, **characterized in that** the anionic polymer is chosen from anionic polymers of grafted silicone type comprising a polysiloxane portion and a portion consisting of a non-silicone organic chain, one of the two portions constituting the main chain of the polymer, the other being grafted onto the said main chain.

7. Composition according to Claim 6, **characterized in that** the grafted silicone polymer is chosen from silicone polymers whose structure comprises the unit of formula (III) below: 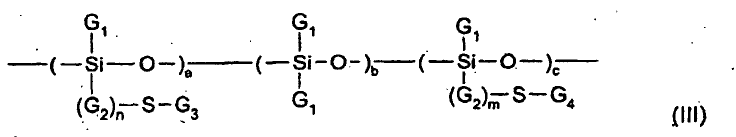 in which the radicals G₁, which may be identical or different, represent hydrogen or a C₁-C₁₀ alkyl radical or alternatively a phenyl radical; the radicals G₂, which may be identical or different, represent a C₁-C₁₀ alkylene group; G₃ represents a polymeric residue resulting from the (homo)polymerization of at least one anionic monomer containing ethylenic unsaturation; G₄ represents a polymeric residue resulting from the (homo)polymerization of at least one hydrophobic monomer containing ethylenic unsaturation; m and n are equal to 0 or 1; a is an integer ranging from 0 to 50; b is an integer which can be between 10 and 350, c is an integer ranging from 0 to 50; with the proviso that one of the parameters a and c is other than 0.

8. Composition according to Claim 7, **characterized in that** the unit of formula (III) has at least one of the following characteristics:
- the radicals G₁ denote a C₁-C₁₀ alkyl radical;
- n is non-zero and the radicals G₂ represent a divalent C₁-C₃ radical;
- G₃ represents a polymeric radical resulting from the (homo) polymerization of at least one monomer such as a carboxylic acid containing ethylenic unsaturation;
- G₄ represents a polymeric radical resulting from the (homo)polymerization of at least one monomer such as a C₁-C₁₀ alkyl (meth)acrylate.

9. Composition according to Claim 7 or 8, **characterized in that** the unit of formula (III) simultaneously has the following characteristics:
- the radicals G₁ denote a methyl radical;
- n is non-zero and the radicals G₂ represent a propylene radical;
- G₃ represents a polymeric radical resulting from the (homo)polymerization of at least acrylic acid and/or methacrylic acid;
- G₄ represents a polymeric radical resulting from the (homo)polymerization of at least isobutyl or methyl (meth)acrylate.

10. Composition according to any one of the preceding claims, **characterized in that** the cationic polymer is chosen from quaternary cellulose ether derivatives, copolymers of cellulose with a watersoluble quaternary ammonium monomer, cyclopolymers, cationic polysaccharides, cationic silicone polymers, quaternized or non-quaternized vinylpyrrolidonedialkylaminoalkyl acrylate or methacrylate copolymers, quaternary polymers of vinylpyrrolidone and of vinylimidazole, and polyaminoamides, and mixtures thereof.

11. Composition according to any one of the preceding claims, **characterized in that** the anionic polymer is a poly(sodium methacrylate). the

12. Composition according to any one of the preceding claims, **characterized in that** the cationic polymer is a hydroxy(C₁-C₄)alkyl cellulose comprising quaternary ammonium groups.

13. Cosmetic composition for coating keratin fibres, comprising at least one film-forming polymer comprising a cationic polymer that is a hydroxy(C1-C4)alkylcellulose containing quaternary ammonium groups and an anionic polymer that is a poly(sodium methacrylate), **characterized in that** it comprises an aqueous dispersion of nonionic film-forming polymer, which is a copolymer of ethyl acrylate and of methyl methacrylate.

14. Composition according to Claim 13, **characterized in that** the cationic polymer is a hydroxyethylcellulose crosslinked with epichlorohydrin quaternized with trimethylamine.

15. Composition according to Claim 13 or 14, **characterized in that** the copolymer of ethyl acrylate and of methyl methacrylate is present in a solids content ranging from 0.1% to 15% and better still from 0.5% to 8% by weight, relative to the total weight of the composition.

16. Composition according to any one of the preceding claims, **characterized in that** the cationic polymer is present in a content ranging from 0.01% to 20% by weight, preferably from 0.01% to 15% by weight and even more preferably from 0.1% to 5% by weight, relative to the total weight of the composition.

17. Composition according to any one of the preceding claims, **characterized in that** the anionic polymer is used in an amount ranging from 0.01 to 20% by weight relative to the total weight of the composition, preferably from 0.05 to 15% by weight and even more preferably from 0.1% to 7% by weight.

18. Composition according to any one of the preceding claims, **characterized in that** it also comprises a wax.

19. Composition according to Claim 18, **characterized in that** the wax is present in a content ranging from 2% to 40% by weight, relative to the total weight of the composition, preferably from 5% to 30% by weight and better still from 10% to 25% by weight.

20. Composition according to either of Claims 18 and 19, **characterized in that** the wax comprises at least one wax (I) having a melting point ranging from 70°C to 110°C.

21. Composition according to Claim 20, **characterized in that** the wax (I) is present in a content ranging from 0.1% to 20% by weight relative to the total weight of the composition.

22. Composition according to any one of the preceding Claims 18 to 21, **characterized in that** the wax comprises at least one wax (II) having a melting point greater than or equal to 45°C and less than 70°C.

23. Composition according to Claim 22, **characterized in that** the wax (II) is present in a wax (I)/wax (II) weight ratio ranging from 0.2 to 1.

24. Composition according to any one of the preceding claims, **characterized in that** it also comprises at least one polyoxyalkylenated silicone.

25. Composition according to any one of the preceding claims, **characterized in that** it comprises at least one dimethicone copolyol.

26. Composition according to any one of the preceding claims, **characterized in that** it also comprises at least one nonionic film-forming polymer other than the copolymer of ethyl acrylate and of methyl methacrylate defined according to either of Claims 1 and 13.

27. Composition according to any one of the preceding claims, **characterized in that** it also comprises water in a content ranging from 1% to 95% by weight relative to the total weight of the composition, and better still from 10 to 80% by weight.

28. Composition according to any one of the preceding claims, **characterized in that** it is in the form of a wax-in-water, water-in-wax, oil-in-water or water-in-oil emulsion.

29. Composition according to any one of the preceding claims, **characterized in that** it also comprises at least one volatile oil.

30. Composition according to any one of the preceding claims, **characterized in that** it comprises at least one emulsifying surfactant. 30,

31. Composition according to Claim 30, **characterized in that** the emulsifying surfactant is present in a content ranging from 2% to 30% by weight relative to the total weight of the composition.

32. Composition according to any one of the preceding claims, **characterized in that** it comprises at least one additive chosen from the group formed by vitamins, trace elements, softeners, sequestering agents, fragrances, oils, thickeners, proteins, ceramides, plasticizers, cohesion agents, basifying or acidifying agents, fillers, pigments, emollients and preserving agents, and mixtures thereof.

33. Composition according to any one of the preceding claims, **characterized in that** the composition is a make-up composition, a make-up base, a composition to be applied over a make-up or a composition for cosmetically treating keratin fibres.

34. Process for coating keratin fibres, in particular the eyelashes, **characterized in that** a composition according to any one of Claims 1 to 33 is applied to the keratin fibres.

35. Use of a composition as defined in any one of Claims 1 to 33, to curl the eyelashes and/or lengthen and/or coat the eyelashes and/or to obtain a fast make-up result and/or a make-up which is easy to apply and/or which has good staying power.
